# EUROPÄISCHE PATENTSCHRIFT

(11) **EP 0 733 626 B1**
(45) Veröffentlichungstag und Bekanntmachung des Hinweises auf die Patenterteilung: **13.01.1999**
(21) Anmeldenummer: 96103608.4
(22) Anmeldetag: 08.03.1996
(51) Int. Cl.: C07D 285/14, C07D 271/12, C07D 513/04, C07D 498/04, A61K 31/41

(54) **N-(Benzofurazanyl)-Arylsulfonamide und ihre Analoge verwendbar als Endothelin-Rezeptor-Antagonisten**
N-(Benzofuranazyl)-arylsulphonamides and their analogues useful as endothelin-receptor-antagonists
N-(Benzofurazanyl)-arylsulfonamides et leurs analogues utiles comme antagonistes du récepteur d'endothéline

(30) Priorität: 18.03.1995 DE 19509950
(43) Veröffentlichungstag der Anmeldung: 25.09.1996
(73) Patentinhaber: MERCK PATENT GmbH, 64293 Darmstadt (DE)
(72) Erfinder: Osswald, Mathias Dr., D-64673 Zwingenberg (DE); Mederski, Werner Dr., D-64390 Erzhausen (DE); Dorsch, Dieter Dr., D-64372 Ober-Ramstadt (DE); Wilm, Claudia Dr., D-64367 Mühltal (DE); Schmitges, Claus Dr., D-64823 Gross-Umstadt (DE); Christadler, Maria, D-63322 Rödermark (DE)

(56) Entgegenhaltungen:
- WO-A-94/27979
- CHEMICAL ABSTRACTS, vol. 71, no. 1, 7.Juli 1969 Columbus, Ohio, US; abstract no. 3332m, V.G. PESIN ET AL.: "2,1,3-Thia- and selenadiazoles. LIII. Amination of benzo-2,1,3-thiadiazole with hydroxylamine sulfate in concentrated sulfuric acid" Seite 312; XP002006432 & KHIM. GETEROTSIKL. SOEDIN, Nr. 5, 1968, Seiten 812-4,
- CHEMICAL ABSTRACTS, vol. 49, no. 5, 10.März 1955 Columbus, Ohio, US; abstract no. 3170a, A.M. KHALETSKII ET AL.: "Synthesis of piazthiole (3,4-benzo-2,1,3-thiadiazole) and its derivatives. II. " XP002006433 & ZHUR. OBSHCHEI KHIM., Bd. 24, 1954, Seiten 133-6,

## Beschreibung

Die Erfindung betrifft Verbindungen der Formel I 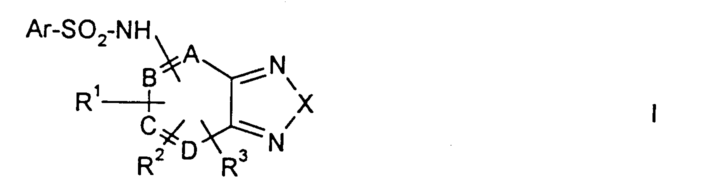 worin
- -A=B-C=D-: eine -CH=CH-CH=CH-Gruppe, in der auch 1 oder 2 CH durch N ersetzt sein können,
- Ar: unsubstituiertes oder ein-, zwei- oder dreifach durch H, Hal, Q, Alkenyl mit bis zu 6 C-Atomen, Ph, OPh, NO₂, NR⁴R⁵, NHCOR⁴, CF₃, OCF₃, CN, OR⁴, COOR⁴, (CH₂)ₙCOOR⁴, (CH₂)ₙNR⁴R⁵, -N=C=O oder NHCONR⁴R⁵ substituiertes Ph oder Naphthyl,
- R¹, R², R³: jeweils unabhängig voneinander fehlen, H, Hal, Q, CF₃, NO₂, NR⁴R⁵, CN, COOR⁴ oder NHCOR⁴,
- R⁴, R⁵: jeweils unabhängig voneinander H oder Q, oder zusammen auch -CH₂-(CH₂)ₙ-CH₂-,
- Q: Alkyl mit 1 bis 6 C-Atomen,
- Ph: Phenyl,
- X: O oder S,
- Hal: F, Cl, Br oder I,
- n: 1, 2 oder 3 bedeuten,
sowie ihre Salze,
ausgenommen

4-Methyl-N-(2,1,3-benzothiadiazol-4-yl)-benzolsulfonamid, 4-Methyl-N-(2,1,3-benzothiadiazol-5-yl)-benzolsulfonamid, 4-Nitro-N-(2,1,3-benzothiadiazol-4-yl)-benzolsulfonamid, 4-Nitro-N-(2,1,3-benzothiadiazol-5-yl)-benzolsulfonamid, 4-Amino-N-(2,1,3-benzothiadiazol-4-yl)-benzolsulfonamid und 4-Amino-N-(2,1,3-benzothiadiazol-5-yl)-benzolsulfonamid.

Ähnliche Verbindungen sind aus EP 0 558 258 A1, EP 0 569 193 A1 und WO 94/27979 bekannt.

4-Methyl-N-(2,1,3-benzothiadiazol-5 (bzw. 4)-yl)-benzolsulfonamid ist in Khim. Geterotsikl. Soedin. (1968), 5, 812-14, 4-Nitro-N-(2,1,3-benzothiadiazol-5 (bzw. 4)-yl)-benzolsulfonamid und 4-Amino-N-(2,1,3-benzothiadiazol-5 (bzw. 4)-yl)-benzolsulfonamid sind in Zh. Obshch. Khim. (1954), 24, 133-136 beschrieben.

Für diese Verbindungen werden aber keine pharmakologischen Wirkungen beschrieben.

Der Erfindung lag die Aufgabe zugrunde, neue Verbindungen mit wertvollen Eigenschatten aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Endothelinrezeptor-antagonistische Eigenschaften und können daher zur Behandlung von Krankheiten wie Hypertonie, Herzinsuffizienz, koronare Herzerkrankung, renale, cerebrale und myocardiale Ischämie, Niereninsuffizienz, Hirninfarkt, subarachnoidale Hämorrhagie, Arteriosklerose, pulmonaler Hochdruck, Entzündungen, Asthma, Prostatahyperplasie, endotoxischer Schock und bei Komplikationen nach der Verabreichung von Substanzen wie z.B. Cyclosporin, sowie anderen, mit Endothelin-Aktivitäten assoziierten Krankheiten eingesetzt werden.

Die Verbindungen zeigen u.a. eine hohe Affinität zu den Endothelin-Subrezeptoren ET_{A} und ET_{B}. Diese Wirkungen können nach üblichen in vitro- oder in vivo-Methoden ermittelt werden, wie z.B. beschrieben von P.D. Stein et al., J. Med. Chem. 37, 1994, 329-331 und E. Ohlstein et al., Proc. Natl. Acad. Sci. USA 91, 1994, 8052-8056.

Die blutdrucksenkende Wirkung wird beschrieben von M.K. Bazil et al., J. Cardiovasc. Pharmacol. 22, 1993, 897-905 und J. Lange et al., Lab Animal 20, 1991, Appl. Note 1016.

Die Verbindungen der Formel I können als Arzneimittelwirkstoffe in der Human- und Veterinärmedizin eingesetzt werden, insbesondere zur Prophylaxe und/oder Therapie von Herz-, Kreislauf- und Gefäßkrankheiten, vor allem von Hypertonie und Herzinsuffizienz.

Gegenstand der Erfindung sind die Verbindungen der Formel I und ihre Salze sowie ein Verfahren zur Herstellung dieser Verbindungen sowie ihrer Salze, dadurch gekennzeichnet, daß man
(a) eine Verbindung der Formel II 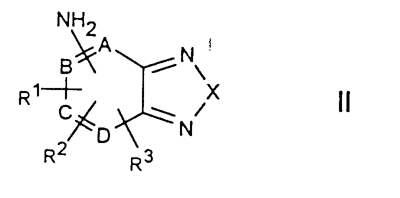 worin
   -A=B-C=D-, R¹, R²,R³ und X die in Anspruch 1 angegebenen Bedeutungen haben,
   mit einer Verbindung der Formel III

      Ar-SO₂-E III
   worin
      - E: Cl, Br, I oder eine freie oder reaktionsfähig funktionell abgewandelte OH-Gruppe bedeutet und
      - Ar: die in Anspruch 1 angegebene Bedeutung hat,
   umsetzt,
   oder
(b) daß man zur Herstellung einer Verbindung der Formel I,
   worin
      - X: S bedeutet
   eine Verbindung der Formel IV 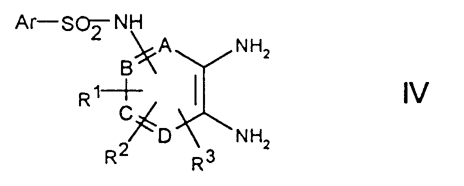
   worin
   -A=B-C=D-, Ar, R¹, R² und R³ die in Anspruch 1 angegebenen Bedeutungen haben,
   mit Thionylchlorid oder einem reaktionsfähigen Derivat dieser Verbindung
   umsetzt,
   oder
c) daß man zur Herstellung einer Verbindung der Formel I,
   worin
      - X: O bedeutet,
   eine Verbindung der Formel V 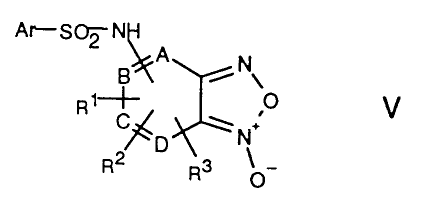
   worin
   -A=B-C=D-, Ar, R¹, R² und R³ die in Anspruch 1 angegebenen Bedeutungen haben,
   reduziert,
   und/oder daß man in einer Verbindung der Formel I einen oder mehrere Rest(e) R¹, R² und/oder R³ in einen oder mehrere andere Reste R¹, R² und/oder R³ umwandelt,
   indem man
      i) eine Nitrogruppe zu einer Aminogruppe reduziert,
      ii) einen Bromsubstituenten durch eine Cyangruppe ersetzt,
      iii) eine Cyangruppe zu einer Carboxygruppe hydrolysiert,

      iv) eine Carboxygruppe verestert,
      v) eine Aminogruppe durch reduktive Aminierung in ein alkyliertes Amin umwandelt oder
      vi) eine Aminogruppe acyliert
   und/oder eine Base oder Säure der Formel I in eines ihrer Salze umwandelt.

Vor- und nachstehend haben die Reste bzw. Parameter -A=B-C=D-, Ar, R¹ bis R⁵, Q, Ph, X, Hal und n die bei den Formeln I bis V angegebenen Bedeutungen, falls nicht ausdrücklich etwas anderes angegeben ist.

In den vorstehenden Formeln hat Q 1 bis 6, vorzugsweise 1, 2, 3 oder 4 C-Atome. Q bedeutet vorzugsweise Methyl, weiterhin Ethyl, Propyl, Isopropyl, Butyl, Isobutyl, sek.-Butyl oder tert.-Butyl, ferner auch Pentyl, 1-, 2-oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

Alkenyl steht vorzugsweise für Vinyl, 1- oder 2-Propenyl, 1-Butenyl, ferner 1-Pentenyl oder 1-Hexenyl.

Hal bedeutet vorzugsweise F, Cl oder Br, aber auch I.

Ar ist unsubstituiertes, vorzugsweise - wie angegeben - monosubstituiertes Phenyl oder Naphthyl, im einzelnen bevorzugt Phenyl, o- oder m-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Phenylphenyl, o-, m- oder p-Hydroxyphenyl, o- oder m-Nitrophenyl, o- oder m-Aminophenyl, o-, m- oder p-Methylaminophenyl, o-, m- oder p-Dimethylaminophenyl, o-, m- oder p-Acetamidophenyl, o-, m- oder p-(Trifluormethoxy)-phenyl, o-, m- oder p-Cyanphenyl, o-, m- oder p-(N-Pyrrolidino)-phenyl, o-, m- oder p-(N-Piperidino)-phenyl, o-, m- oder p-Methoxyphenyl, o-, m- oder p-Ethoxyphenyl, o-, m- oder p-Phenoxyphenyl, o-, m- oder p-Carboxyphenyl, o-, m- oder p-Methoxycarbonylphenyl, o-, m- oder p- Ethoxycarbonylphenyl, o-, m- oder p-(Carboxymethyl)-phenyl, o-, m- oder p- (Methoxycarbonylmethyl)-phenyl, o-, m- oder p-(Methoxycarbonyl-ethyl)-phenyl, o-, m- oder p-(Aminomethyl)-phenyl, o-, m- oder p-(N-Methylaminomethyl)-phenyl, o-, m- oder p-(N,N-Dimethylamino)-phenyl, o-, m- oder p-(N-Ethylamino)-phenyl-, o-, m- oder p-(N,N-Diethylamino)-phenyl, o-, m- oder p-(N,N-Dimethylaminomethyl)-phenyl, o-, m- oder p-(N-Pyrrolidino-methyl)-phenyl, o-, m- oder p-(N-Piperidinomethyl)-phenyl, o-, m- oder p-Isocyanatophenyl, o-, m- oder p-Carbamidophenyl, o-, m- oder p-(N-Methylcarbamoyl)-phenyl, o-, m- oder p-(N,N-Dimethylcarbamoyl)-phenyl, o-, m- oder p-Fluorphenyl, o-, m- oder p-Bromphenyl, o-, m- oder p- Chlorphenyl, weiter bevorzugt Naphthyl, 5-Methyl-naphthyl, 5-Ethylnaphthyl, 5-Propylnaphthyl, 5-Isopropylnaphthyl, 5-tert.-Butylnaphthyl, 5-Trifluormethylnaphthyl, 5-Phenylnaphthyl, 5-Hydroxynaphthyl, 5-Nitronaphthyl, 5-Aminonaphthyl, 5-N-Methylaminonaphthyl, 5-N,N-Dimethylaminonaphthyl, 5-Acetamidonaphthyl, 5-(Trifluormethoxy)-naphthyl, 5-Cyannaphthyl, 5-(N-Pyrrolidino)-naphthyl, 5-(N-Piperidino)-naphthyl, 5-Methoxynaphthyl, 5-Ethoxynaphthyl, 5-Carboxynaphthyl, 5-Methoxycarbonylnaphthyl, 5-Ethoxycarbonylnaphthyl, 5-(Carboxymethyl)-naphthyl, 5-(Methoxycarbonylmethyl)-naphthyl, 5-(Ethoxycarbonylmethyl)-naphthyl, 5-(Aminomethyl)-naphthyl, 5-(N-Methylaminomethyl)-naphthyl, 5-(N,N-Dimethylaminomethyl)-naphthyl, 5-(N-Ethylaminonaphthyl, 5-(N,N-Diethylamino)-naphthyl, 5-(N-Pyrrolidinomethyl)-naphthyl, 5-(N-Piperidinomethyl)-naphthyl, 5-Isocyanatonaphthyl, 5-Carbamidonaphthyl, 5-(N-Methylcarbamoyl)-naphthyl, 5-(N,N-Dimethylcarbamoyl)-naphthyl, 5-N-Isopropylaminonaphthyl, 5-N-Isopropyl-N-methylaminonaphthyl, 5-Fluornaphthyl, 5-Chlornaphthyl, 5-Bromnaphthyl, weiter bevorzugt 2,3-, 2,4-, 2,5-, 2,6-, 3,4- oder 3,5-Difluorphenyl, 2,3-, 2,4-, 2,5-, 2,6-, 3,4-oder 3,5-Dichlorphenyl, 2,3-, 2,4-, 2,5-, 2,6-, 3,4- oder 3,5-Dibromphenyl, 2-Chlor-3-methyl-, 2-Chlor-4-methyl-, 2-Chlor-5-methyl-, 2-Chlor-6-methyl-, 2-Methyl-3-chlor-, 2-Methyl-4-chlor-, 2-Methyl-5-chlor-, 2-Methyl-6-chlor-, 3-Chlor-4-methyl-, 3-Chlor-5-methyl- oder 3-Methyl-4-chlorphenyl, 2-Brom-3-methyl-, 2-Brom-4-methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyl- oder 3-Methyl-4-bromphenyl, 2,4- oder 2,5-Dinitrophenyl, 2,5- oder 3,4-Dimethoxyphenyl, 3-Nitro-4-chlorphenyl, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 3-Carboxy-2-methoxy-, 3-Carboxy-4-methoxy- oder 3-Carboxy-5-Methoxyphenyl, 2,3,4-, 2,3,5-, 2,3,6-, 2,4,6- oder 3,4,5-Trichlorphenyl, 2,4,6-tri-tert.-Butylphenyl, ferner bevorzugt 2-Nitro-4-(trifluormethyl)phenyl, 3,5-di-(trifluormethyl)-phenyl, 2,5-dimethylphenyl, 2-Hydroxy-3,5-dichlorphenyl, 2-Fluor-5- oder 4-Fluor-3-(trifluormethyl)-phenyl, 4-Chlor-2- oder 4-Chlor-3-(trifluormethyl)-, 2-Chlor-4- oder 2-Chlor-5-(trifluormethyl)-phenyl, 4-Brom-2- oder 4-Brom-3-(trifluormethyl)-phenyl, p-Iodphenyl, p-Vinylphenyl, 5-(N,N-Dibutylamino)-naphthyl, 2-Nitro-4-methoxyphenyl, 2,5-Dimethoxy-4-nitrophenyl, 3,5-Dicarboxyphenyl, 2-Chlor-3-nitro-5-carboxy-phenyl, 4-Chlor-3-carboxyphenyl, 2-Methyl-5-nitrophenyl, 2,4-dimethyl-3-nitrophenyl, 3,6-Dichlor-4-Aminophenyl, 4-Fluor-3-chlorphenyl, 4-Fluor-3,5-dimethylphenyl, 2-Fluor-4-Bromphenyl, 2,5-Difluor-4-bromphenyl, 2,4-Dichlor-5-methylphenyl, 3-Brom-6-methoxyphenyl, 3-Chlor-6-methoxyphenyl, 3-Chlor-4-acetamidophenyl, 4-Hydroxy-3-carboxyphenyl, 2-Methoxy-5-methylphenyl oder 2,4,6-Triisopropylphenyl.

Der Rest -A=B-C=D- steht bevorzugt für -CH=CH-CH=N-, ferner für -CH=N-CH=CH- oder -CH=N-CH=N-, besonders bevorzugt aber für -CH=CH-CH=CH-.

Die Reste R¹, R² und R³ bedeuten jeweils unabhängig voneinander vorzugsweise H, Q (insbesondere CH₃, Hal, insbesondere Chlor oder Brom, ferner aber auch bevorzugt NO₂ oder CF₃.

Der Parameter n ist vorzgsweise 0 oder 1, ferner bevorzugt 2.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzgten Bedeutungen hat. Einige bevorzugte Gruppen von Verbindungen können durch die folgenden Teilformeln Ia bis Ig ausgedrückt werden, die der Formel I entsprechen und worin die nicht näher bezeichneten Reste, die bei der Formel I angegebene Bedeutung haben, worin jedoch

| | | |
|---|---|---|
| in Ia | X | S bedeutet; |
| in Ib | X | O bedeutet; |
| in Ic | X | S und |
| | -A=B-C=D- | -CH=CH-CH=CH- bedeuten; |
| in Id | X | O und |
| | -A=B-C=D- | -CH=CH-CH=CH- bedeuten; |
| in Ie | X | O und |
| | -A=B-C=D- | -CH=CH-CH=N- bedeuten; |
| in If | X | S, |
| | -A=B-C=D- | -CH=CH-CH=CH-, |
| | R¹ | H, |
| | R² | Hal und |
| | R³ | Methyl bedeuten; |
| in Ig | Ar | 5-(N,N-Dimethylamino)-naphthyl bedeutet. |

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in der EP 0569 193 A1 und WO 94/27979) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können vorzugsweise erhalten werden, indem man Verbindungen der Formel II mit Verbindungen der Formel III umsetzt.

In den Verbindungen der Formel III bedeutet E vorzugsweise Cl, Br, I oder eine reaktionsfähig abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung wird in der Regel in einem inerten Lösungsmittel in Gegenwart einer oder mehrerer Base(n), vorzugsweise eines tertiären Amins, z.B. Triethylamin, Pyridin, 4-Dimethylaminopyridin vorgenommen, zweckmäßig bei Temperaturen zwischen 0 und 150°, vorzugsweise zwischen 40 und 90°. Ein Überschuß des Amins kann auch als Lösungsmittel dienen.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl-oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Ausgangsverbindungen der Formel II sind in der Regel neu. Sie können aber nach an sich bekannten Methoden hergestellt werden. So kann z.B. 5-Amino-6-methyl-2,1,3-benzothiadiazol durch Hydrierung an Raney-Nickel in einem inerten Lösungsmittel wie Methanol aus 5-Nitro-6-methyl-2,1,3-benzothiadiazol hergestellt werden Dies erfolgt zweckmäßig bei Temperaturen zwischen 0 und etwa 200°; vorzugsweise arbeitet man zwischen 30 und 80°.

Verbindungen der Formel I, worin X S bedeutet, können weiterhin erhalten werden, indem man Verbindungen der Formel IV, die in der Regel neu sind, mit Thionylchlorid oder einem reaktionsfähigen Derivat dieser Verbindung wie z.B. Thionylanilin, analog bekannter Verfahren, wie sie in der Literatur (z.B. J. Heterocycl. Chem. 7, 629 (1970)) beschrieben sind, in einem inerten Lösungsmittel in Gegenwart einer oder mehrerer Base(n), vorzugsweise eines tertiären Amins, z.B. Triethylamin, Pyridin, 4-Dimethylaminopyridin umsetzt, zweckmäßig bei Temperaturen zwischen 0 und 150°, vorzugsweise zwischen 40 und 90°. Ein Überschuß des Amins kann auch als Lösungsmittel dienen.

Verbindungen der Formel I, worin X O bedeutet, können weiterhin erhalten werden, indem man Verbindungen der Formel V, die in der Regel neu sind, mit reduzierenden Verbindungen wie Cl-SO₂-N=C=O, POCl₃, PCl₃, Na₂S₂O₄, Ph₃P oder P(OC₂H₅)₃ analog bekannter Verfahren, wie sie in der Literatur (z. B. Tetrahedron 44, 5209 (1988), Tetrahedron 48, 8199 (1992), Z.Chem. 20, 257 (1980), J. Org. Chem. 47, 1774 (1982), J. Org. Chem. 28, 1656 (1963) oder J. Med. Chem. 11, 305 (1968)) beschrieben sind, umsetzt.

Die Ausgangsverbindungen der Formel V können nach an sich bekannten Methoden hergestellt werden. So kann z.B. 4-tert.-Butyl-N-(2,1,3-benzoxadiazol-1-N-oxid-5-yl)-benzolsulfonamid durch Umsetzung mit Natriumazid unter Phasentransferkatalyse aus 4-tert.-Butyl-N-(4-chlor-3-nitro)-benzolsulfonamid über 4-tert.-Butyl-N-(4-azido-3-nitro)-benzolsulfonamid und anschließende Cyclisierung in Eisessig hergestellt werden.

Es ist ferner möglich, eine Verbindung der Formel I in eine andere Verbindung der Formel I umzuwandeln, indem man einen oder mehrere Rest(e) R¹, R² und/oder R³ in einen oder mehrere andere Reste R¹, R² und/oder R³ umwandelt, z.B. indem man
Nitrogruppen, beispielsweise durch Hydrierung an Raney-Nickel oder Pd-Kohle in einem inerten Lösungsmittel wie Methanol oder Ethanol, zu Aminogruppen reduziert und/oder
Bromsubstituenten durch Umsetzung mit z.B. Kupfer-I-cyanid in Cyangruppen umwandelt und/oder
Cyangruppen zu COOH-Gruppen hydrolysiert und/oder
Carboxygruppen durch Umsetzung mit Alkoholen verestert und/oder
Nitrogruppen unter hydrogenolytischen Bedingungen alkyliert, wobei alkylierte Amine erhalten werden.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Gewünschtenfalls kann in einer Verbindung der Formel I eine funktionell abgewandelte Amino- und /oder Hydroxygruppe durch Solvolyse oder Hydrogenolyse nach üblichen Methoden in Freiheit gesetzt werden. So kann z.B. eine Verbindung der Formel I, die eine NHCOR⁴- oder eine COOR⁴-Gruppe enthält, in die entsprechende Verbindung der Formel I umgewandelt werden, die stattdessen eine NH₂- oder eine COOH-Gruppe enthält. COOR⁴-Gruppen können z.B. mit NaOH oder KOH in Wasser, Wasser-THF oder Wasser-Dioxan bei Temperaturen zwischen 0 und 100° verseift werden.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono und disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium-oder Kaliumhydroxid oder -carbonat) in die entsprechenden Metall-, insbesondere Alkalimetall- oder Erdalkalimetall-, oder in die entsprechenden Ammoniumsalze umgewandelt werden.

Gegenstand der Erfindung ist ferner die Verwendung der Verbindungen der Formel I und/oder 4-Methyl-N-(2,1,3-benzothiadiazol-5-yl)-benzolsulfonamid und/oder 4-Nitro-N-(2,1,3-benzothiadiazol-5-yl)-benzolsulfonamid und/oder 4-Amino-N-(2,1,3-benzothiadiazol-5-yl)-benzolsulfonamid und/oder ihrer physiologisch unbedenklichen Salze zur Herstellung pharmazeutischer Zubereitungen, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder 4-Methyl-N-(2,1,3-benzothiadiazol-5-yl)-benzolsulfonamid und/oder 4-Nitro-N-(2,1,3-benzothiadiazol-5-yl)-benzolsulfonamid und/oder 4-Amino-N-(2,1,3-benzothiadiazol-5-yl)-benzolsulfonamid und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks-, Aroma- und/oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, insbesondere von Hypertonie und Herzinsuffizienz verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

### Beispiel 1

Zu einer Lösung von 1,53 g 5-Amino-2,1,3-benzothiadiazol, erhältlich durch Hydrierung von 5-Nitro-2,1,3-benzothiadiazol an Raney-Nickel in Methanol, in 15 ml Pyridin gibt man eine Lösung von 3 g 5-Dimethylaminonaphthalin-sulfochlorid ("A") in 10 ml Pyridin, rührt das Gemisch 24 Stunden bei 60°, gibt es anschließend auf 75 ml 2N Salzsäure und arbeitet wie üblich auf. Man erhält 5-Dimethylamino-N-(2,1,3-benzothiadiazol-5-yl)-1-naphthalinsulfonamid als gelben Feststoff, F.73°, Kaliumsalz, F. > 300°.

Analog erhält man aus folgenden mZ-5-Amino-2,1,3-benzothiadiazolen, worin mZ
4-Methyl
6-Methyl
7-Methyl
4,6-Dimethyl
4,7-Dimethyl
6,7-Dimethyl
4-Trifluormethyl
6-Trifluormethyl
7-Trifluormethyl
4-Brom
6-Brom
7-Brom
4,6-Dibrom
4,7-Dibrom
6,7-Dibrom
4-Brom-6-methyl
4-Brom-7-methyl
6-Brom-7-methyl
4-Methyl-6-brom
4-Methyl-7-brom
6-Methyl-7-brom
4-Brom-6-ethyl
4-Brom-7-ethyl
6-Brom-7-ethyl
4-Ethyl-6-brom
4-Ethyl-7-brom
6-Ethyl-7-brom
4-Brom-6-trifluormethyl
4-Brom-7-trifluormethyl
6-Brom-7-trifluormethyl
4-Trifluormethyl-6-brom
4-Trifluormethyl-7-brom
6-Trifluormethyl-7-brom
4-Chlor
6-Chlor
7-Chlor
4-Nitro
6-Nitro
7-Nitro
4-Brom-6-tert.-butyl
4-Brom-7-tert.-butyl
6-Brom-7-tert.-butyl
4-tert.-Butyl-6-brom
4-tert.-Butyl-7-brom
6-tert.-Butyl-7-brom
4-Chlor-6-methyl
4-Chlor-7-methyl
6-Chlor-7-methyl
4-Methyl-6-chlor
4-Methyl-7-chlor
6-Methyl-7-chlor
4-Dimethylamino
6-Dimethylamino
7-Dimethylamino
4-Cyan
6-Cyan
7-Cyan
4-Methoxycarbonyl
6-Methoxycarbonyl
7-Methoxycarbonyl
4-Ethoxycarbonyl
6-Ethoxycarbonyl
7-Ethoxycarbonyl
4-Acetamido
6-Acetamido
7-Acetamido
bedeutet
mit "A" die nachstehenden 5-Dimethylamino-mZ-1-naphthalinsulfonamide, worin mZ
N-(4-methyl-2,1,3-benzothiadiazol-5-yl), F. 159°
N-(6-methyl-2,1,3-benzothiadiazol-5-yl)
N-(7-methyl-2,1,3-benzothiadiazol-5-yl)
N-(4,6-dimethyl-2,1,3-benzothiadiazol-5-yl)
N-(4,7-dimethyl-2,1,3-benzothiadiazol-5-yl)
N-(6,7-dimethyl-2,1,3-benzothiadiazol-5-yl)
N-(4-trifluormethyl-2,1,3-benzothiadiazol-5-yl)
N-(6-trifluormethyl-2,1,3-benzothiadiazol-5-yl)
N-(7-trifluormethyl-2,1,3-benzothiadiazol-5-yl)
N-(4-brom-2,1,3-benzothiadiazol-5-yl), F. 151 °
N-(6-brom-2,1,3-benzothiadiazol-5-yl)
N-(7-brom-2,1,3-benzothiadiazol-5-yl)
N-(4,6-dibrom-2,1,3-benzothiadiazol-5-yl)
N-(4,7-dibrom-2,1,3-benzothiadiazol-5-yl)
N-(6,7-dibrom-2,1,3-benzothiadiazol-5-yl)
N-(4-brom-6-methyl-2,1,3-benzothiadiazol-5-yl)
N-(4-brom-7-methyl-2,1,3-benzothiadiazol-5-yl)
N-(6-brom-7-methyl-2,1,3-benzothiadiazol-5-yl)
N-(4-methyl-6-brom-2,1,3-benzothiadiazol-5-yl)
N-(4-methyl-7-brom-2,1,3-benzothiadiazol-5-yl)
N-(6-methyl-7-brom-2,1,3-benzothiadiazol-5-yl)
N-(4-brom-6-ethyl-2,1,3-benzothiadiazol-5-yl)
N-(4-brom-7-ethyl-2,1,3-benzothiadiazol-5-yl)
N-(6-brom-7-ethyl-2,1,3-benzothiadiazol-5-yl)
N-(4-ethyl-6-brom-2,1,3-benzothiadiazol-5-yl)
N-(4-ethyl-7-brom-2,1,3-benzothiadiazol-5-yl)
N-(6-ethyl-7-brom-2,1,3-benzothiadiazol-5-yl)
N-(4-brom-6-trifluormethyl-2,1,3-benzothiadiazol-5-yl)
N-(4-brom-7-trifluormethyl-2,1,3-benzothiadiazol-5-yl)
N-(6-brom-7-trifluormethyl-2,1,3-benzothiadiazol-5-yl)
N-(4-trifluormethyl-6-brom-2,1,3-benzothiadiazol-5-yl)
N-(4-trifluormethyl-7-brom-2,1,3-benzothiadiazol-5-yl)
N-(6-trifluormethyl-7-brom-2,1,3-benzothiadiazol-5-yl)
N-(4-chlor-2,1,3-benzothiadiazol-5-yl), F. 168°
N-(6-chlor-2,1,3-benzothiadiazol-5-yl)
N-(7-chlor-2,1,3-benzothiadiazol-5-yl)
N-(4-nitro-2,1,3-benzothiadiazol-5-yl)
N-(6-nitro-2,1,3-benzothiadiazol-5-yl)
N-(7-nitro-2,1,3-benzothiadiazol-5-yl)
N-(4-bromo-6-tert.-butyl-2,1,3-benzothiadiazol-5-yl)
N-(4-brom-7-tert.-butyl-2,1,3-benzothiadiazol-5-yl)
N-(6-brom-7-tert.-butyl-2,1,3-benzothiadiazol-5-yl)
N-(4-tert.-butyl-6-brom-2,1,3-benzothiadiazol-5-yl)
N-(4-tert.-butyl-7-brom-2,1,3-benzothiadiazol-5-yl)
N-(6-tert.-butyl-7-brom-2,1,3-benzothiadiazol-5-yl)
N-(4-chloro-6-methyl-2,1,3-benzothiadiazol-5-yl)
N-(4-chlor-7-methyl-2,1,3-benzothiadiazol-5-yl)
N-(6-chlor-7-methyl-2,1,3-benzothiadiazol-5-yl)
N-(4-methyl-6-chlor-2,1,3-benzothiadiazol-5-yl)
N-(4-methyl-7-chlor-2,1,3-benzothiadiazol-5-yl)
N-(6-methyl-7-chlor-2,1,3-benzothiadiazol-5-yl)
N-(4-dimethylamino-2,1,3-benzothiadiazol-5-yl)
N-(6-dimethylamino-2,1,3-benzothiadiazol-5-yl)
N-(7-dimethylamino-2,1,3-benzothiadiazol-5-yl)
N-(4-cyan-2,1,3-benzothiadiazol-5-yl)
N-(6-cyan-2,1,3-benzothiadiazol-5-yl)
N-(7-cyan-2,1,3-benzothiadiazol-5-yl)
N-(4-methoxycarbonyl-2,1,3-benzothiadiazol-5-yl)
N-(6-methoxycarbonyl-2,1,3-benzothiadiazol-5-yl)
N-(7-methoxycarbonyl-2,1,3-benzothiadiazol-5-yl)
N-(4-ethoxycarbonyl-2,1,3-benzothiadiazol-5-yl)
N-(6-ethoxycarbonyl-2,1,3-benzothiadiazol-5-yl)
N-(7-ethoxycarbonyl-2,1,3-benzothiadiazol-5-yl)
N-(4-acetamido-2,1,3-benzothiadiazol-5-yl)
N-(6-acetamido-2,1,3-benzothiadiazol-5-yl)
N-(7-acetamido-2,1,3-benzothiadiazol-5-yl)
bedeutet.

Analog erhält man durch Umsetzung von 4-Amino-2,1,3-benzothiadiazol mit "A" 5-Dimethylamino-N-(2,1,3-benzothiadiazol-4-yl)-1-naphthalinsulfonamid, F. 81°
und aus den folgenden 4-Amino-mZ-2,1,3-benzothiadiazolen, worin mZ
5-Methyl
6-Methyl
7-Methyl
5,6-Dimethyl
5,7-Dimethyl
6,7-Dimethyl
5-Trifluormethyl
6-Trifluormethyl
7-Trifluormethyl
5-Brom
6-Brom
7-Brom
5,6-Dibrom
5,7-Dibrom
6,7-Dibrom
5-Brom-6-methyl
5-Brom-7-methyl
6-Brom-7-methyl
5-Methyl-6-brom
5-Methyl-7-brom
6-Methyl-7-brom
5-Brom-6-ethyl
5-Brom-7-ethyl
6-Brom-7-ethyl
5-Ethyl-6-brom
5-Ethyl-7-brom
6-Ethyl-7-brom
5-Brom-6-trifluormethyl
5-Brom-7-trifluormethyl
6-Brom-7-trifluormethyl
5-Trifluormethyl-6-brom
5-Trifluormethyl-7-brom
6-Trifluormethyl-7-brom
5-Chlor
6-Chlor
7-Chlor
5-Nitro
6-Nitro
7-Nitro
5-Brom-6-tert.-butyl
5-Brom-7-tert.-butyl
6-Brom-7-tert.-butyl
5-tert.-Butyl-6-brom
5-tert.-Butyl-7-brom
6-tert.-Butyl-7-brom
5-Chlor-6-methyl
5-Chlor-7-methyl
6-Chlor-7-methyl
5-Methyl-6-chlor
5-Methyl-7-chlor
6-Methyl-7-chlor
5-Dimethylamino
6-Dimethylamino
7-Dimethylamino
5-Cyan
6-Cyan
7-Cyan
5-Methoxycarbonyl
6-Methoxycarbonyl
7-Methoxycarbonyl
5-Ethoxycarbonyl
6-Ethoxycarbonyl
7-Ethoxycarbonyl
5-Acetamino
6-Acetamido
7-Acetamido
bedeutet
mit "A" die nachstehenden 5-Dimethylamino-mZ-1-naphthalinsulfonamide, worin mZ
N-(5-methyl-2,1,3-benzothiadiazol-4-yl), F .154°
N-(6-methyl-2,1,3-benzothiadiazol-4-yl)
N-(7-methyl-2,1,3-benzothiadiazol-4-yl), F. 170°
N-(5,6-dimethyl-2,1,3-benzothiadiazol-4-yl)
N-(5,7-dimethyl-2,1,3-benzothiadiazol-4-yl)
N-(6,7-dimethyl-2,1,3-benzothiadiazol-4-yl)
N-(5-trifluormethyl-2,1,3-benzothiadiazol-4-yl), F. 182°
N-(6-trifluormethyl-2,1,3-benzothiadiazol-4-yl)
N-(7-trifluormethyl-2,1,3-benzothiadiazol-4-yl)
N-(5-brom-2,1,3-benzothiadiazol-4-yl)
N-(6-brom-2,1,3-benzothiadiazol-4-yl)
N-(7-brom-2,1,3-benzothiadiazol-4-yl)
N-(5,6-dibrom-2,1,3-benzothiadiazol-4-yl)
N-(5,7-dibrom-2,1,3-benzothiadiazol-4-yl)
N-(6,7-dibrom-2,1,3-benzothiadiazol-4-yl)
N-(5-brom-6-methyl-2,1,3-benzothiadiazol-4-yl)
N-(5-brom-7-methyl-2,1,3-benzothiadiazol-4-yl)
N-(6-brom-7-methyl-2,1,3-benzothiadiazol-4-yl)
N-(5-methyl-6-brom-2,1,3-benzothiadiazol-4-yl)
N-(5-methyl-7-brom-2,1,3-benzothiadiazol-4-yl)
N-(6-methyl-7-brom-2,1,3-benzothiadiazol-4-yl)
N-(5-brom-6-ethyl-2,1,3-benzothiadiazol-4-yl)
N-(5-brom-7-ethyl-2,1,3-benzothiadiazol-4-yl)
N-(6-brom-7-ethyl-2,1,3-benzothiadiazol-4-yl)
N-(5-ethyl-6-brom-2,1,3-benzothiadiazol-4-yl)
N-(5-ethyl-7-brom-2,1,3-benzothiadiazol-4-yl)
N-(6-ethyl-7-brom-2,1,3-benzothiadiazol-4-yl)
N-(5-brom-6-trifluormethyl-2,1,3-benzothiadiazol-4-yl)
N-(5-brom-7-trifluormethyl-2,1,3-benzothiadiazol-4-yl)
N-(6-brom-7-trifluormethyl-2,1,3-benzothiadiazol-4-yl)
N-(5-trifluormethyl-6-brom-2,1,3-benzothiadiazol-4-yl)
N-(5-trifluormethyl-7-brom-2,1,3-benzothiadiazol-4-yl)
N-(6-trifluormethyl-7-brom-2,1,3-benzothiadiazol-4-yl)
N-(5-chlor-2,1,3-benzothiadiazol-4-yl)
N-(6-chlor-2,1,3-benzothiadiazol-4-yl)
N-(7-chlor-2,1,3-benzothiadiazol-4-yl)
N-(5-nitro-2,1,3-benzothiadiazol-4-yl)
N-(6-nitro-2,1,3-benzothiadiazol-4-yl)
N-(7-nitro-2,1,3-benzothiadiazol-4-yl)
N-(5-brom-6-tert.-butyl-2,1,3-benzothiadiazol-4-yl)
N-(5-brom-7-tert.-butyl-2,1,3-benzothiadiazol-4-yl)
N-(6-brom-7-tert.-butyl-2,1,3-benzothiadiazol-4-yl)
N-(5-tert.-butyl-6-brom-2,1,3-benzothiadiazol-4-yl)
N-(5-tert.-butyl-7-brom-2,1,3-benzothiadiazol-4-yl)
N-(6-tert.-butyl-7-brom-2,1,3-benzothiadiazol-4-yl)
N-(5-chlor-6-methyl-2,1,3-benzothiadiazol-4-yl)
N-(5-chlor-7-methyl-2,1,3-benzothiadiazol-4-yl)
N-(6-chlor-7-methyl-2,1,3-benzothiadiazol-4-yl), F. 170°
N-(5-methyl-6-chlor-2,1,3-benzothiadiazol-4-yl)
N-(5-methyl-7-chlor-2,1,3-benzothiadiazol-4-yl)
N-(6-methyl-7-chlor-2,1,3-benzothiadiazol-4-yl)
N-(5-dimethylamino-2,1,3-benzothiadiazol-4-yl)
N-(6-dimethylamino-2,1,3-benzothiadiazol-4-yl)
N-(7-dimethylamino-2,1,3-benzothiadiazol-4-yl)
N-(5-cyan-2,1,3-benzothiadiazol-4-yl)
N-(6-cyan-2,1,3-benzothiadiazol-4-yl)
N-(7-cyan-2,1,3-benzothiadiazol-4-yl)
N-(5-methoxycarbonyl-2,1,3-benzothiadiazol-4-yl)
N-(6-methoxycarbonyl-2,1,3-benzothiadiazol-4-yl)
N-(7-methoxycarbonyl-2,1,3-benzothiadiazol-4-yl)
N-(5-ethoxycarbonyl-2,1,3-benzothiadiazol-4-yl)
N-(6-ethoxycarbonyl-2,1,3-benzothiadiazol-4-yl)
N-(7-ethoxycarbonyl-2,1,3-benzothiadiazol-4-yl)
N-(5-acetamido-2,1,3-benzothiadiazol-4-yl)
N-(6-acetamido-2,1,3-benzothiadiazol-4-yl)
N-(7-acetamido-2,1,3-benzothiadiazol-4-yl)
bedeutet.

### Beispiel 2

Zu einer Lösung von 3,01 g 4-tert.-Butyl-N-(1,2-diamino-4-phenyl)-1-benzolsulfonamid und 4,1 g Triethylamin in 100 ml Toluol gibt man eine Lösung von 3 g Thionylchlorid in 15 ml Toluol, erhitzt das Gemisch eine Stunde bei 110°, arbeitet wie üblich auf und erhält 4-tert.-Butyl-N-(2,1,3-benzothiadiazol-5-yl)-1-benzolsulfonamid, F. 198°.

Analog erhält man durch Umsetzung von N-(1,2-Diamino-4-phenyl)-1-benzolsulfonamid mit Thionylchlorid N-(2,1,3-benzothiadiazol-5-yl)-1-benzolsulfonamid und durch analoge Umsetzung folgender N-(1,2-Diamino-4-phenyl)-mZ-1-benzolsulfonamide, worin mZ
4-Fluor
4-Chlor
4-Brom
4-Iod
4-Ethyl
4-Propyl
4-Isopropyl
3,4-Dimethyl
2,5-Dimethyl
2,5-Diethyl
2,4-Diethyl
2,5-Dipropyl
3-Acetamido
4-Acetamido
2-Cyan
3-Carboxy
2-Trifluormethoxy
3-Nitro
4-Nitro
2-Nitro-5-methyl
2-Methyl-5-nitro
2-Ethyl-5-nitro
2,4,6-Trimethyl
2,4-Dichlor
2,5-Dichlor
3,4-Dichlor
3,5-Dichlor
5-Brom-2-methoxy
5-Brom-2-methyl
5-Brom-2-ethyl
5-Brom-2-propyl
2,5-Difluor
3,6-Difluor
2,5-Dimethoxy
3,4-Dimethoxy
3-Trifluormethyl
4-Trifluormethyl
3,5-Di-(trifluormethyl)
2,4,5-Trichlor
2-Chlor-4-fluor
3-Chlor-5-fluor
2-Chlor-5-methyl
3-Chlor-2-methyl
5-Chlor-2-methoxy
2-Butyl-5-Brom
2-Brom-5-butyl
2-Brom-5-propyl
5-Fluor-2-methyl
2-Phenyl
2-Phenyl
4-tolyl
2-Brom-5-ethyl
bedeutet,
mit Thionylchlorid die nachstehenden N-(2,1,3-benzothiadiazol-5-yl)-mZ-1-benzolsulfonamide,
worin mZ
4-Fluor
4-Chlor
4-Brom, F. 195°
4-Iod
4-Ethyl
4-Propyl
4-Isopropyl
3,4-Dimethyl
2,5-Dimethyl
2,5-Diethyl
2,4-Diethyl
2,5-Dipropyl
3-Acetamido
4-Acetamido
2-Cyan
3-Carboxy
2-Trifluormethoxy
3-Nitro
4-Nitro
2-Nitro-5-methyl
2-Methyl-5-nitro
2-Ethyl-5-nitro
2,4,6-Trimethyl
2,4-Dichlor
2,5-Dichlor
3,4-Dichlor
3,5-Dichlor
5-Brom-2-methoxy
5-Brom-2-methyl, F. 185°
5-Brom-2-ethyl
5-Brom-2-propyl, F. 182°
2,5-Difluor
3,6-Difluor
2,5-Dimethoxy
3,4-Dimethoxy
3-Trifluormethyl
4-Trifluormethyl
3,5-Di-(trifluormethyl)
2,4,5-Trichlor
2-Chlor-4-fluor
3-Chlor-5-fluor
2-Chlor-5-methyl
3-Chlor-2-methyl
5-Chlor-2-methoxy
2-Butyl-5-Brom
2-Brom-5-butyl
2-Brom-5-Propyl, F. 299°
5-Fluor-2-methyl
2-Phenyl, F. 175°
4-Tolyl, F. 220°
2-Brom-5-ethyl, F. 183°
bedeutet.

### Beispiel 3

Eine Lösung von 1 g 5-Dimethylamino-N-(2,1,3-benzoxadiazol-5-yl-1- oder 3-N-oxid)-1-naphthalinsulfonamid (erhältlich durch Erhitzen von 5-Dimethylamino-N-(1-azido-2-nitro-4-phenyl)-1-naphthalinsulfonamid in Eisessig) und 5 ml Triethylphosphit in 50 ml absolutem Ethanol wird 30 Minuten bei 75° erhitzt. Nach Entfernung des Lösungsmittels und üblicher Aufarbeitung erhält man 5-Dimethylamino-N-(2,1,3-benzoxadiazol-5-yl)-1-naphthalinsulfonamid, F. 106°

Analog erhält man durch Reaktion folgender 5-Dimethylamino-mZ-1-naphthalinsulfonamide,
worin mZ
N-(4-Methyl-2,1,3-benzoxadiazol-5-yl-1- oder 3-N-oxid)
N-(6-Methyl-2,1,3-benzoxadiazol-5-yl-1- oder 3-N-oxid)
N-(7-Methyl-2,1,3-benzoxadiazol-5-yl-1- oder 3-N-oxid)
N-(4,6-Dimethyl-2,1,3-benzoxadiazol-5-yl-1- oder 3-N-oxid)
N-(4,7-Dimethyl-2,1,3-benzoxadiazol-5-yl-1- oder 3-N-oxid)
N-(6,7-Dimethyl-2,1,3-benzoxadiazol-5-yl-1- oder 3-N-oxid)
N-(4-Trifluormethyl-2,1,3-benzoxadiazol-5-yl-1- oder 3-N-oxid)
N-(6-Trifluormethyl-2,1,3-benzoxadiazol-5-yl-1- oder 3-N-oxid)
N-(7-Trifluormethyl-2,1,3-benzoxadiazol-5-yl-1- oder 3-N-oxid)
N-(4-Brom-2,1,3-benzoxadiazol-5-yl-1- oder 3-N-oxid)
N-(6-Brom-2,1,3-benzoxadiazol-5-yl-1- oder 3-N-oxid)
N-(7-Brom-2,1,3-benzoxadiazol-5-yl-1- oder 3-N-oxid)
N-(4,6-Dibrom-2,1,3-benzoxadiazol-5-yl-1- oder 3-N-oxid)
N-(4,7-Dibrom-2,1,3-benzoxadiazol-5-yl-1- oder 3-N-oxid)
N-(6,7-Dibrom-2,1,3-benzoxadiazol-5-yl-1- oder 3-N-oxid)
N-(4-Brom-6-methyl-2,1,3-benzoxadiazol-5-yl-1- oder 3-N-oxid)
N-(4-Brom-7-methyl-2,1,3-benzoxadiazol-5-yl-1- oder 3-N-oxid)
N-(6-Brom-7-methyl-2,1,3-benzoxadiazol-5-yl-1- oder 3-N-oxid)
N-(4-Methyl-6-brom-2,1,3-benzoxadiazol-5-yl-1- oder 3-N-oxid)
N-(4-Methyl-7-brom-2,1,3-benzoxadiazol-5-yl-1- oder 3-N-oxid)
N-(6-Methyl-7-brom-2,1,3-benzoxadiazol-5-yl-1- oder 3-N-oxid)
N-(4-Brom-6-ethyl-2,1,3-benzoxadiazol-5-yl-1- oder 3-N-oxid)
N-(4-Brom-7-ethyl-2,1,3-benzoxadiazol-5-yl-1- oder 3-N-oxid)
N-(6-Brom-7-ethyl-2,1,3-benzoxadiazol-5-yl-1- oder 3-N-oxid)
N-(4-Ethyl-6-brom-2,1,3-benzoxadiazol-5-yl-1- oder 3-N-oxid)
N-(4-Ethyl-7-brom-2,1,3-benzoxadiazol-5-yl-1- oder 3-N-oxid)
N-(6-Ethyl-7-brom-2,1,3-benzoxadiazol-5-yl-1- oder 3-N-oxid)
N-(4-Brom-6-trifluormethyl-2,1,3-benzoxadiazol-5-yl-1- oder 3-N-oxid)
N-(4-Brom-7-trifluormethyl-2,1,3-benzoxadiazol-5-yl-1- oder 3-N-oxid)
N-(6-Brom-7-trifluormethyl-2,1,3-benzoxadiazol-5-yl-1- oder 3-N-oxid)
N-(4-Trifluormethyl-6-brom-2,1,3-benzoxadiazol-5-yl-1- oder 3-N-oxid)
N-4-Trifluormethyl-7-brom-2,1,3-benzoxadiazol-5-yl-1- oder 3-N-oxid)
N-(6-Trifluormethyl-7-brom-2,1,3-benzoxadiazol-5-yl-1- oder 3-N-oxid)
N-(4-Chlor-2,1,3-benzoxadiazol-5-yl-1- oder 3-N-oxid)
N-(6-Chlor-2,1,3-benzoxadiazol-5-yl-1- oder 3-N-oxid)
N-(7-Chlor-2,1,3-benzoxadiazol-5-yl-1- oder 3-N-oxid)
N-(4-Nitro-2,1,3-benzoxadiazol-5-yl-1- oder 3-N-oxid)
N-(6-Nitro-2,1,3-benzoxadiazol-5-yl-1- oder 3-N-oxid)
N-(7-Nitro-2,1,3-benzoxadiazol-5-yl-1- oder 3-N-oxid)
N-(4-Brom-6-tert.-butyl-2,1,3-benzoxadiazol-5-yl-1- oder 3-N-oxid)
N-(4-Brom-7-tert.-butyl-2,1,3-benzoxadiazol-5-yl-1- oder 3-N-oxid)
N-(6-Brom-7-tert.-butyl-2,1,3-benzoxadiazol-5-yl-1- oder 3-N-oxid)
N-(4-tert.-Butyl-6-brom-2,1,3-benzoxadiazol-5-yl-1- oder 3-N-oxid)
N-(4-tert.-Butyl-7-brom-2,1,3-benzoxadiazol-5-yl-1- oder 3-N-oxid)
N-(6-tert.-Butyl-7-brom-2,1,3-benzoxadiazol-5-yl-1- oder 3-N-oxid)
N-(4-Chlor-6-methyl-2,1,3-benzoxadiazol-5-yl-1- oder 3-N-oxid)
N-(4-Chlor-7-methyl-2,1,3-benzoxadiazol-5-yl-1- oder 3-N-oxid)
N-(6-Chlor-7-methyl-2,1,3-benzoxadiazol-5-yl-1- oder 3-N-oxid)
N-(4-Methyl-6-chlor-2,1,3-benzoxadiazol-5-yl-1- oder 3-N-oxid)
N-(4-Methyl-7-chlor-2,1,3-benzoxadiazol-5-yl-1- oder 3-N-oxid)
N-(6-Methyl-7-chlor-2,1,3-benzoxadiazol-5-yl-1- oder 3-N-oxid)
N-(4-Dimethylamino-2,1,3-benzoxadiazol-5-yl-1- oder 3-N-oxid)
N-(6-Dimethylamino-2,1,3-benzoxadiazol-5-yl-1- oder 3-N-oxid)
N-(7-Dimethylamino-2,1,3-benzoxadiazol-5-yl-1- oder 3-N-oxid)
N-(4-Cyan-2,1,3-benzoxadiazol-5-yl-1- oder 3-N-oxid)
N-(6-Cyan-2,1,3-benzoxadiazol-5-yl-1- oder 3-N-oxid)
N-(7-Cyan-2,1,3-benzoxadiazol-5-yl-1- oder 3-N-oxid)
N-(4-Methoxycarbonyl-2,1,3-benzoxadiazol-5-yl-1- oder 3-N-oxid)
N-(6-Methoxycarbonyl-2,1,3-benzoxadiazol-5-yl-1- oder 3-N-oxid)
N-(7-Methoxycarbonyl-2,1,3-benzoxadiazol-5-yl-1- oder 3-N-oxid)
N-(4-Ethoxycarbonyl-2,1,3-benzoxadiazol-5-yl-1- oder 3-N-oxid)
N-(6-Ethoxycarbonyl-2,1,3-benzoxadiazol-5-yl-1- oder 3-N-oxid)
N-(7-Ethoxycarbonyl-2,1,3-benzoxadiazol-5-yl-1- oder 3-N-oxid)
N-(4-Acetamino-2,1,3-benzoxadiazol-5-yl-1- oder 3-N-oxid)
N-(6-Acetamido-2,1,3-benzoxadiazol-5-yl-1- oder 3-N-oxid)
N-(7-Acetamido-2,1,3-benzoxadiazol-5-yl-1- oder 3-N-oxid)
bedeutet
mit Triethylphosphit die nachstehenden 5-Dimethylamino-mZ-1-naphthalinsulfonamide,
worin mZ
N-(4-methyl-2,1,3-benzoxadiazol-5-yl)
N-(6-methyl-2,1,3-benzoxadiazol-5-yl)
N-(7-methyl-2,1,3-benzoxadiazol-5-yl)
N-(4,6-dimethyl-2,1,3-benzoxadiazol-5-yl)
N-(4,7-dimethyl-2,1,3-benzoxadiazol-5-yl)
N-(6,7-dimethyl-2,1,3-benzoxadiazol-5-yl)
N-(4-trifluormethyl-2,1,3-benzoxadiazol-5-yl)
N-(6-trifluormethyl-2,1,3-benzoxadiazol-5-yl)
N-(7-trifluormethyl-2,1,3-benzoxadiazol-5-yl)
N-(4-brom-2,1,3-benzoxadiazol-5-yl)
N-(6-brom-2,1,3-benzoxadiazol-5-yl)
N-(7-brom-2,1,3-benzoxadiazol-5-yl)
N-(4,6-dibrom-2,1,3-benzoxadiazol-5-yl)
N-(4,7-dibrom-2,1,3-benzoxadiazol-5-yl)
N-(6,7-dibrom-2,1,3-benzoxadiazol-5-yl)
N-(4-brom-6-methyl-2,1,3-benzoxadiazol-5-yl)
N-(4-brom-7-methyl-2,1,3-benzoxadiazol-5-yl)
N-(6-brom-7-methyl-2,1,3-benzoxadiazol-5-yl)
N-(4-methyl-6-brom-2,1,3-benzoxadiazol-5-yl)
N-(4-methyl-7-brom-2,1,3-benzoxadiazol-5-yl)
N-(6-methyl-7-brom-2,1,3-benzoxadiazol-5-yl)
N-(4-brom-6-ethyl-2,1,3-benzoxadiazol-5-yl)
N-(4-brom-7-ethyl-2,1,3-benzoxadiazol-5-yl)
N-(6-brom-7-ethyl-2,1,3-benzoxadiazol-5-yl)
N-(4-ethyl-6-brom-2,1,3-benzoxadiazol-5-yl)
N-(4-ethyl-7-brom-2,1,3-benzoxadiazol-5-yl)
N-(6-ethyl-7-brom-2,1,3-benzoxadiazol-5-yl)
N-(4-brom-6-trifluormethyl-2,1,3-benzoxadiazol-5-yl)
N-(4-brom-7-trifluormethyl-2,1,3-benzoxadiazol-5-yl)
N-(6-brom-7-trifluormethyl-2,1,3-benzoxadiazol-5-yl)
N-(4-trifluormethyl-6-brom-2,1,3-benzoxadiazol-5-yl)
N-(4-trifluormethyl-7-brom-2,1,3-benzoxadiazol-5-yl)
N-(6-trifluormethyl-7-brom-2,1,3-benzoxadiazol-5-yl)
N-(4-chlor-2,1,3-benzoxadiazol-5-yl)
N-(6-chlor-2,1,3-benzoxadiazol-5-yl)
N-(7-chlor-2,1,3-benzoxadiazol-5-yl)
N-(4-nitro-2,1,3-benzoxadiazol-5-yl)
N-(6-nitro-2,1,3-benzoxadiazol-5-yl)
N-(7-nitro-2,1,3-benzoxadiazol-5-yl)
N-(4-brom-6-tert.-butyl-2,1,3-benzoxadiazol-5-yl)
N-(4-brom-7-tert.-butyl-2,1,3-benzoxadiazol-5-yl)
N-(6-brom-7-tert.-butyl-2,1,3-benzoxadiazol-5-yl)
N-(4-tert.-butyl-6-brom-2,1,3-benzoxadiazol-5-yl)
N-(4-tert.-butyl-7-brom-2,1,3-benzoxadiazol-5-yl)
N-(6-tert.-butyl-7-brom-2,1,3-benzothiadiazol-5-yl)
N-(4-chlor-6-methyl-2,1,3-benzoxadiazol-5-yl)
N-(4-chlor-7-methyl-2,1,3-benzoxadiazol-5-yl)
N-(6-chlor-7-methyl-2,1,3-benzoxadiazol-5-yl)
N-(4-methyl-6-chlor-2,1,3-benzoxadiazol-5-yl)
N-(4-methyl-7-chlor-2,1,3-benzoxadiazol-5-yl)
N-(6-methyl-7-chlor-2,1,3-benzoxadiazol-5-yl)
N-(4-dimethylamino-2,1,3-benzoxadiazol-5-yl)
N-(6-dimethylamino-2,1,3-benzoxadiazol-5-yl)
N-(7-dimethylamino-2,1,3-benzoxadiazol-5-yl)
N-(4-cyan-2,1,3-benzoxadiazol-5-yl)
N-(6-cyan-2,1,3-benzoxadiazol-5-yl)
N-(7-cyan-2,1,3-benzoxadiazol-5-yl)
N-(4-methoxycarbonyl-2,1,3-benzoxadiazol-5-yl)
N-(6-methoxycarbonyl-2,1,3-benzoxadiazol-5-yl)
N-(7-methoxycarbonyl-2,1,3-benzoxadiazol-5-yl)
N-(4-ethoxycarbonyl-2,1,3-benzoxadiazol-5-yl)
N-(6-ethoxycarbonyl-2,1,3-benzoxadiazol-5-yl)
N-(7-ethoxycarbonyl-2,1,3-benzoxadiazol-5-yl)
N-(4-acetamido-2,1,3-benzoxadiazol-5-yl)
N-(6-acetamido-2,1,3-benzoxadiazol-5-yl)
N-(7-acetamido-2,1,3-benzoxadiazol-5-yl)
bedeutet.

### Beispiel 4

Eine Lösung von 1 g 3-Nitro-N-(2,1,3-benzothiadiazol-5-yl)-1-benzolsulfonamid in 25 ml Methanol wird bei Normaldruck und 20° bis zum Stillstand an 1 g Raney-Nickel hydriert. Man filtriert, dampft ein und erhält 3-Amino-N-(2,1,3-benzothiadiazol-5-yl)-1-benzolsulfonamid.

Analog erhält man aus
4-Nitro-N-(2,1,3-benzothiadiazol-5-yl)-1-benzolsulfonamid
2-Nitro-5-methyl-N-(2,1,3-benzothiadiazol-5-yl)-1-benzolsulfonamid
2-Methyl-5-nitro-N-(2,1,3-benzothiadiazol-5-yl)-1-benzolsulfonamid
4-Nitro-N-(2,1,3-benzoxadiazol-5-yl)-1-benzolsulfonamid
3-Nitro-N-(2,1,3-benzoxadiazol-5-yl)-1-benzolsulfonamid
2-Nitro-5-methyl-N-(2,1,3-benzoxadiazol-5-yl)-1-benzolsulfonamid
2-Methyl-5-Nitro-N-(2,1,3-benzoxadiazol-5-yl)-1-benzolsulfonamid
die nachstehenden Verbindungen
4-Amino-N-(2,1,3-benzothiadiazol-5-yl)-1-benzolsulfonamid
2-Amino-5-methyl-N-(2,1,3-benzothiadiazol-5-yl)-1-benzolsulfonamid
2-Amino-5-nitro-N-(2,1,3-benzothiadiazol-5-yl)-1-benzolsulfonamid
4-Amino-N-(2,1,3-benzoxadiazol-5-yl)-1-benzolsulfonamid
3-Amino-N-(2,1,3-benzoxadiazol-5-yl)-1-benzolsulfonamid
2-Amino-5-methyl-N-(2,1,3-benzoxadiazol-5-yl)-1-benzolsulfonamid
2-Methyl-5-Amino-N-(2,1,3-benzoxadiazol-5-yl)-1-benzolsulfonamid.

### Beispiel 5

Analog Beispiel 1 erhält man durch Umsetzung von 5-Amino-2,1,3-benzoxadiazol (erhältlich durch Reduktion von 5-Nitro-2,1,3-benzoxadiazol-1- oder 3-N-oxid mit Triethylphosphit) mit 2-Ethylbenzolsulfochlorid das 2-Ethyl-N-(2,1,3-benzoxadiazol-5-yl)-1-benzolsulfonamid.

Analog erhält man aus den 5-Amino-mZ-2,1,3-benzoxadiazolen,
worin mZ
4-Methyl
6-Methyl
7-Methyl
4,6-Dimethyl
4,7-Dimethyl
6,7-Dimethyl
4-Trifluormethyl
6-Trifluormethyl
7-Trifluormethyl
4-Brom
6-Brom
7-Brom
4,6-Dibrom
4,7-Dibrom
6,7-Dibrom
4-Brom-6-methyl
4-Brom-7-methyl
6-Brom-7-methyl
4-Methyl-6-brom
4-Methyl-7-brom
6-Methyl-7-brom
4-Brom-6-ethyl
4-Brom-7-ethyl
6-Brom-7-ethyl
4-Ethyl-6-brom
4-Ethyl-7-brom
6-Ethyl-7-brom
4-Brom-6-trifluormethyl
4-Brom-7-trifluormethyl
6-Brom-7-trifluormethyl
4-Trifluormethyl-6-brom
4-Trifluormethyl-7-brom
6-Trifluormethyl-7-brom
4-Chlor
6-Chlor
7-Chlor
4-Nitro
6-Nitro
7-Nitro
4-Brom-6-tert.-butyl
4-Brom-7-tert.-butyl
6-Brom-7-tert.-butyl
4-tert.-Butyl-6-brom
4-tert.-Butyl-7-brom
6-tert.-Butyl-7-brom
4-Chlor-6-methyl
4-Chlor-7-methyl
6-Chlor-7-methyl
4-Methyl-6-chlor
4-Methyl-7-chlor
6-Methyl-7-chlor
4-Dimethylamino
6-Dimethylamino
7-Dimethylamino
4-Cyan
6-Cyan
7-Cyan
4-Methoxycarbonyl
6-Methoxycarbonyl
7-Methoxycarbonyl
4-Ethoxycarbonyl
6-Ethoxycarbonyl
7-Ethoxycarbonyl
4-Acetamido
6-Acetamido
7-Acetamido
bedeutet
durch Umsetzung mit 2-Ethyl-benzolsulfochlorid die nachstehenden 2-Ethyl-mZ-1-benzolsulfonamide,
worin mZ
N-(4-methyl-2,1,3-benzoxadiazol-5-yl)
N-(6-methyl-2,1,3-benzoxadiazol-5-yl)
N-(7-methyl-2,1,3-benzoxadiazol-5-yl)
N-(4,6-dimethyl-2,1,3-benzoxadiazol-5-yl)
N-(4,7-dimethyl-2,1,3-benzoxadiazol-5-yl)
N-(6,7-dimethyl-2,1,3-benzoxadiazol-5-yl)
N-(4-trifluormethyl-2,1,3-benzoxadiazol-5-yl)
N-(6-trifluormethyl-2,1,3-benzoxadiazol-5-yl)
N-(7-trifluormethyl-2,1,3-benzoxadiazol-5-yl)
N-(4-brom-2,1,3-benzoxadiazol-5-yl)
N-(6-brom-2,1,3-benzoxadiazol-5-yl)
N-(7-brom-2,1,3-benzoxadiazol-5-yl)
N-(4,6-dibrom-2,1,3-benzoxadiazol-5-yl)
N-(4,7-dibrom-2,1,3-benzoxadiazol-5-yl)
N-(6,7-dibrom-2,1,3-benzoxadiazol-5-yl)
N-(4-brom-6-methyl-2,1,3-benzoxadiazol-5-yl)
N-(4-brom-7-methyl-2,1,3-benzoxadiazol-5-yl)
N-(6-brom-7-methyl-2,1,3-benzoxadiazol-5-yl)
N-(4-methyl-6-brom-2,1,3-benzoxadiazol-5-yl)
N-(4-methyl-7-brom-2,1,3-benzoxadiazol-5-yl)
N-(6-methyl-7-brom-2,1,3-benzoxadiazol-5-yl)
N-(4-brom-6-ethyl-2,1,3-benzoxadiazol-5-yl)
N-(4-brom-7-ethyl-2,1,3-benzoxadiazol-5-yl)
N-(6-brom-7-ethyl-2,1,3-benzoxadiazol-5-yl)
N-(4-ethyl-6-brom-2,1,3-benzoxadiazol-5-yl)
N-(4-ethyl-7-brom-2,1,3-benzoxadiazol-5-yl)
N-(6-ethyl-7-brom-2,1,3-benzoxadiazol-5-yl)
N-(4-brom-6-trifluormethyl-2,1,3-benzoxadiazol-5-yl)
N-(4-brom-7-trifluormethyl-2,1,3-benzoxadiazol-5-yl)
N-(6-brom-7-trifluormethyl-2,1,3-benzoxadiazol-5-yl)
N-(4-trifluormethyl-6-brom-2,1,3,-benzoxadiazol-5-yl)
N-(4-trifluormethyl-7-brom-2,1,3-benzoxadiazol-5-yl)
N-(6-trifluormethyl-7-brom-2,1,3-benzoxadiazol-5-yl)
N-(4-chlor-2,1,3-benzoxadiazol-5-yl)
N-(6-chlor-2,1,3-benzoxadiazol-5-yl)
N-(7-chlor-2,1,3-benzoxadiazol-5-yl)
N-(4-nitro-2,1,3-benzoxadiazol-5-yl)
N-(6-nitro-2,1,3-benzoxadiazol-5-yl)
N-(7-nitro-2,1,3-benzoxadiazol-5-yl)
N-(4-brom-6-tert.-butyl-2,1,3-benzoxadiazol-5-yl)
N-(4-brom-7-tert.-butyl-2,1,3-benzoxadiazol-5-yl)
N-(6-brom-7-tert.-butyl-2,1,3-benzoxadiazol-5-yl)
N-(4-tert.-butyl-6-brom-2,1,3-benzoxadiazol-5-yl)
N-(4-tert.-butyl-7-brom-2,1,3-benzoxadiazol-5-yl)
N-(6-tert.-butyl-7-brom-2,1,3-benzothiadiazol-5-yl)
N-(4-chlor-6-methyl-2,1,3-benzoxadiazol-5-yl)
N-(4-chlor-7-methyl-2,1,3-benzoxadiazol-5-yl)
N-(6-chlor-7-methyl-2,1,3-benzoxadiazol-5-yl)
N-(4-methyl-6-chlor-2,1,3-benzoxadiazol-5-yl)
N-(4-methyl-7-chlor-2,1,3-benzoxadiazol-5-yl)
N-(6-methyl-7-chlor-2,1,3-benzoxadiazol-5-yl)
N-(4-dimethylamino-2,1,3-benzoxadiazol-5-yl)
N-(6-dimethylamino-2,1,3-benzoxadiazol-5-yl)
N-(7-dimethylamino-2,1,3-benzoxadiazol-5-yl)
N-(4-cyan-2,1,3-benzoxadiazol-5-yl)
N-(6-cyan-2,1,3-benzoxadiazol-5-yl)
N-(7-cyan-2,1,3-benzoxadiazol-5-yl)
N-(4-methoxycarbonyl-2,1,3-benzoxadiazol-5-yl)
N-(6-methoxycarbonyl-2,1,3-benzoxadiazol-5-yl)
N-(7-methoxycarbonyl-2,1,3-benzoxadiazol-5-yl)
N-(4-ethoxycarbonyl-2,1,3-benzoxadiazol-5-yl)
N-(6-ethoxycarbonyl-2,1,3-benzoxadiazol-5-yl)
N-(7-ethoxycarbonyl-2,1,3-benzoxadiazol-5-yl)
N-(4-acetamido-2,1,3-benzoxadiazol-5-yl)
N-(6-acetamido-2,1,3-benzoxadiazol-5-yl)
N-(7-acetamido-2,1,3-benzoxadiazol-5-yl)
bedeutet.

Durch entsprechende Umsetzungen mit 2-Ethyl-5-brom-benzolsulfochlorid erhält man die nachstehenden 2-Ethyl-5-brom-mZ-1-benzolsulfonamide,
worin mZ
N-(4-methyl-2,1,3-benzoxadiazol-5-yl)
N-(6-methyl-2,1,3-benzoxadiazol-5-yl)
N-(7-methyl-2,1,3-benzoxadiazol-5-yl)
N-(4,6-dimethyl-2,1,3-benzoxadiazol-5-yl)
N-(4,7-dimethyl-2,1,3-benzoxadiazol-5-yl)
N-(6,7-dimethyl-2,1,3-benzoxadiazol-5-yl)
N-(4-trifluormethyl-2,1,3-benzoxadiazol-5-yl)
N-(6-trifluormethyl-2,1,3-benzoxadiazol-5-yl)
N-(7-trifluormethyl-2,1,3-benzoxadiazol-5-yl)
N-(4-brom-2,1,3-benzoxadiazol-5-yl)
N-(6-brom-2,1,3-benzoxadiazol-5-yl)
N-(7-brom-2,1,3-benzoxadiazol-5-yl)
N-(4,6-dibrom-2,1,3-benzoxadiazol-5-yl)
N-(4,7-dibrom-2,1,3-benzoxadiazol-5-yl)
N-(6,7-dibrom-2,1,3-benzoxadiazol-5-yl)
N-(4-brom-6-methyl-2,1,3-benzoxadiazol-5-yl)
N-(4-brom-7-methyl-2,1,3-benzoxadiazol-5-yl)
N-(6-brom-7-methyl-2,1,3-benzoxadiazol-5-yl)
N-(4-methyl-6-brom-2,1,3-benzoxadiazol-5-yl)
N-(4-methyl-7-brom-2,1,3-benzoxadiazol-5-yl)
N-(6-methyl-7-brom-2,1,3-benzoxadiazol-5-yl)
N-(4-brom-6-ethyl-2,1,3-benzoxadiazol-5-yl)
N-(4-brom-7-ethyl-2,1,3-benzoxadiazol-5-yl)
N-(6-brom-7-ethyl-2,1,3-benzoxadiazol-5-yl)
N-(4-ethyl-6-brom-2,1,3-benzoxadiazol-5-yl)
N-(4-ethyl-7-brom-2,1,3-benzoxadiazol-5-yl)
N-(6-ethyl-7-brom-2,1,3-benzoxadiazol-5-yl)
N-(4-brom-6-trifluormethyl-2,1,3-benzoxadiazol-5-yl)
N-(4-brom-7-trifluormethyl-2,1,3-benzoxadiazol-5-yl)
N-(6-brom-7-trifluormethyl-2,1,3-benzoxadiazol-5-yl)
N-(4-trifluormethyl-6-brom-2,1,3-benzoxadiazol-5-yl)
N-(4-trifluormethyl-7-brom-2,1,3-benzoxadiazol-5-yl)
N-(6-trifluormethyl-7-brom-2,1,3-benzoxadiazol-5-yl)
N-(4-chlor-2,1,3-benzoxadiazol-5-yl)
N-(6-chlor-2,1,3-benzoxadiazol-5-yl)
N-(7-chlor-2,1,3-benzoxadiazol-5-yl)
N-(4-nitro-2,1,3-benzoxadiazol-5-yl)
N-(6-nitro-2,1,3-benzoxadiazol-5-yl)
N-(7-nitro-2,1,3-benzoxadiazol-5-yl)
N-(4-brom-6-tert.-butyl-2,1,3-benzoxadiazol-5-yl)
N-(4-brom-7-tert.-butyl-2,1,3-benzoxadiazol-5-yl)
N-(6-brom-7-tert.-butyl-2,1,3-benzoxadiazol-5-yl)
N-(4-tert.-butyl-6-brom-2,1,3-benzoxadiazol-5-yl)
N-(4-tert.-butyl-7-brom-2,1,3-benzoxadiazol-5-yl)
N-(6-tert.-butyl-7-brom-2,1,3-benzothiadiazol-5-yl)
N-(4-chlor-6-methyl-2,1,3-benzoxadiazol-5-yl)
N-(4-chlor-7-methyl-2,1,3-benzoxadiazol-5-yl)
N-(6-chlor-7-methyl-2,1,3-benzoxadiazol-5-yl)
N-(4-methyl-6-chlor-2,1,3-benzoxadiazol-5-yl)
N-(4-methyl-7-chlor-2,1,3-benzoxadiazol-5-yl)
N-(6-methyl-7-chlor-2,1,3-benzoxadiazol-5-yl)
N-(4-dimethylamino-2,1,3-benzoxadiazol-5-yl)
N-(6-dimethylamino-2,1,3-benzoxadiazol-5-yl)
N-(7-dimethylamino-2,1,3-benzoxadiazol-5-yl)
N-(4-cyan-2,1,3-benzoxadiazol-5-yl)
N-(6-cyan-2,1,3-benzoxadiazol-5-yl)
N-(7-cyan-2,1,3-benzoxadiazol-5-yl)
N-(4-methoxycarbonyl-2,1,3-benzoxadiazol-5-yl)
N-(6-methoxycarbonyl-2,1,3-benzoxadiazol-5-yl)
N-(7-methoxycarbonyl-2,1,3-benzoxadiazol-5-yl)
N-(4-ethoxycarbonyl-2,1,3-benzoxadiazol-5-yl)
N-(6-ethoxycarbonyl-2,1,3-benzoxadiazol-5-yl)
N-(7-ethoxycarbonyl-2,1,3-benzoxadiazol-5-yl)
N-(4-acetamido-2,1,3-benzoxadiazol-5-yl)
N-(6-acetamido-2,1,3-benzoxadiazol-5-yl)
N-(7-acetamido-2,1,3-benzoxadiazol-5-yl)
bedeutet,
sowie durch analoge Umsetzung mit 2-Propyl-5-brom-benzolsulfochlorid erhält man die nachstehenden 2-Propyl-5-brom-mZ-1-benzolsulfonamide, worin mZ
N-(4-methyl-2,1,3-benzoxadiazol-5-yl)
N-(6-methyl-2,1,3-benzoxadiazol-5-yl)
N-(7-methyl-2,1,3-benzoxadiazol-5-yl)
N-(4,6-dimethyl-2,1,3-benzoxadiazol-5-yl)
N-(4,7-dimethyl-2,1,3-benzoxadiazol-5-yl)
N-(6,7-dimethyl-2,1,3-benzoxadiazol-5-yl)
N-(4-trifluormethyl-2,1,3-benzoxadiazol-5-yl)
N-(6-trifluormethyl-2,1,3-benzoxadiazol-5-yl)
N-(7-trifluormethyl-2,1,3-benzoxadiazol-5-yl)
N-(4-brom-2,1,3-benzoxadiazol-5-yl)
N-(6-brom-2,1,3-benzoxadiazol-5-yl)
N-(7-brom-2,1,3-benzoxadiazol-5-yl)
N-(4,6-dibrom-2,1,3-benzoxadiazol-5-yl)
N-(4,7-dibrom-2,1,3-benzoxadiazol-5-yl)
N-(6,7-dibrom-2,1,3-benzoxadiazol-5-yl)
N-(4-brom-6-methyl-2,1,3-benzoxadiazol-5-yl)
N-(4-brom-7-methyl-2,1,3-benzoxadiazol-5-yl)
N-(6-brom-7-methyl-2,1,3-benzoxadiazol-5-yl)
N-(4-methyl-6-brom-2,1,3-benzoxadiazol-5-yl)
N-(4-methyl-7-brom-2,1,3-benzoxadiazol-5-yl)
N-(6-methyl-7-brom-2,1,3-benzoxadiazol-5-yl)
N-(4-brom-6-ethyl-2,1,3-benzoxadiazol-5-yl)
N-(4-brom-7-ethyl-2,1,3-benzoxadiazol-5-yl)
N-(6-brom-7-ethyl-2,1,3-benzoxadiazol-5-yl)
N-(4-ethyl-6-brom-2,1,3-benzoxadiazol-5-yl)
N-(4-ethyl-7-brom-2,1,3-benzoxadiazol-5-yl)
N-(6-ethyl-7-brom-2,1,3-benzoxadiazol-5-yl)
N-(4-brom-6-trifluormethyl-2,1,3-benzoxadiazol-5-yl)
N-(4-brom-7-trifluormethyl-2,1,3-benzoxadiazol-5-yl)
N-(6-brom-7-trifluormethyl-2,1,3-benzoxadiazol-5-yl)
N-(4-trifluormethyl-6-brom-2,1,3-benzoxadiazol-5-yl)
N-(4-trifluormethyl-7-brom-2,1,3-benzoxadiazol-5-yl)
N-(6-trifluormethyl-7-brom-2,1,3-benzoxadiazol-5-yl)
N-(4-chlor-2,1,3-benzoxadiazol-5-yl)
N-(6-chlor-2,1,3-benzoxadiazol-5-yl)
N-(7-chlor-2,1,3-benzoxadiazol-5-yl)
N-(4-nitro-2,1,3-benzoxadiazol-5-yl)
N-(6-nitro-2,1,3-benzoxadiazol-5-yl)
N-(7-nitro-2,1,3-benzoxadiazol-5-yl)
N-(4-brom-6-tert.-butyl-2,1,3-benzoxadiazol-5-yl)
N-(4-brom-7-tert.-butyl-2,1,3-benzoxadiazol-5-yl)
N-(6-brom-7-tert.-butyl-2,1,3-benzoxadiazol-5-yl)
N-(4-tert.-butyl-6-brom-2,1,3-benzoxadiazol-5-yl)
N-(4-tert.-butyl-7-brom-2,1,3-benzoxadiazol-5-yl)
N-(6-tert.-butyl-7-brom-2,1,3-benzothiadiazol-5-yl)
N-(4-chlor-6-methyl-2,1,3-benzoxadiazol-5-yl)
N-(4-chlor-7-methyl-2,1,3-benzoxadiazol-5-yl)
N-(6-chlor-7-methyl-2,1,3-benzoxadiazol-5-yl)
N-(4-methyl-6-chlor-2,1,3-benzoxadiazol-5-yl)
N-(4-methyl-7-chlor-2,1,3-benzoxadiazol-5-yl)
N-(6-methyl-7-chlor-2,1,3-benzoxadiazol-5-yl)
N-(4-dimethylamino-2,1,3-benzoxadiazol-5-yl)
N-(6-dimethylamino-2,1,3-benzoxadiazol-5-yl)
N-(7-dimethylamino-2,1,3-benzoxadiazol-5-yl)
N-(4-cyan-2,1,3-benzoxadiazol-5-yl)
N-(6-cyan-2,1,3-benzoxadiazol-5-yl)
N-(7-cyan-2,1,3-benzoxadiazol-5-yl)
N-(4-methoxycarbonyl-2,1,3-benzoxadiazol-5-yl)
N-(6-methoxycarbonyl-2,1,3-benzoxadiazol-5-yl)
N-(7-methoxycarbonyl-2,1,3-benzoxadiazol-5-yl)
N-(4-ethoxycarbonyl-2,1,3-benzoxadiazol-5-yl)
N-(6-ethoxycarbonyl-2,1,3-benzoxadiazol-5-yl)
N-(7-ethoxycarbonyl-2,1,3-benzoxadiazol-5-yl)
N-(4-acetamido-2,1,3-benzoxadiazol-5-yl)
N-(6-acetamido-2,1,3-benzoxadiazol-5-yl)
N-(7-acetamido-2,1,3-benzoxadiazol-5-yl)
bedeutet.

Analog erhält man aus den 5-Amino-mZ-2,1,3-benzothiadiazolen, worin mZ
4-Methyl
6-Methyl
7-Methyl
4,6-Dimethyl
4,7-Dimethyl
6,7-Dimethyl
4-Trifluormethyl
6-Trifluormethyl
7-Trifluormethyl
4-Brom
6-Brom
7-Brom
4,6-Dibrom
4,7-Dibrom
6,7-Dibrom
4-Brom-6-methyl
4-Brom-7-methyl
6-Brom-7-methyl
4-Methyl-6-brom
4-Methyl-7-brom
6-Methyl-7-brom
4-Brom-6-ethyl
4-Brom-7-ethyl
6-Brom-7-ethyl
4-Ethyl-6-brom
4-Ethyl-7-brom
6-Ethyl-7-brom
4-Brom-6-trifluormethyl
4-Brom-7-trifluormethyl
6-Brom-7-trifluormethyl
4-Trifluormethyl-6-brom
4-Trifluormethyl-7-brom
6-Trifluormethyl-7-brom
4-Chlor
6-Chlor
7-Chlor
4-Nitro
6-Nitro
7-Nitro
4-Brom-6-tert.-butyl
4-Brom-7-tert.-butyl
6-Brom-7-tert.-butyl
4-tert.-Butyl-6-brom
4-tert.-Butyl-7-brom
6-tert.-Butyl-7-brom
4-Chlor-6-methyl
4-Chlor-7-methyl
6-Chlor-7-methyl
4-Methyl-6-chlor
4-Methyl-7-chlor
6-Methyl-7-chlor
4-Dimethylamino
6-Dimethylamino
7-Dimethylamino
4-Cyan
6-Cyan
7-Cyan
4-Methoxycarbonyl
6-Methoxycarbonyl
7-Methoxycarbonyl
4-Ethoxycarbonyl
6-Ethoxycarbonyl
7-Ethoxycarbonyl
4-Acetamido
6-Acetamido
7-Acetamido
bedeutet
durch Umsetzung mit 2-Ethyl-benzolsulfochlorid die nachstehenden 2-Ethyl-mZ-1-benzolsulfonamide,
worin mZ
N-(4-methyl-2,1,3-benzothiadiazol-5-yl)
N-(6-methyl-2,1,3-benzothiadiazol-5-yl)
N-(7-methyl-2,1,3-benzothiadiazol-5-yl)
N-(4,6-dimethyl-2,1,3-benzothiadiazol-5-yl)
N-(4,7-dimethyl-2,1,3-benzothiadiazol-5-yl)
N-(6,7-dimethyl-2,1,3-benzothiadiazol-5-yl)
N-(4-trifluormethyl-2,1,3-benzothiadiazol-5-yl)
N-(6-trifluormethyl-2,1,3-benzothiadiazol-5-yl)
N-(7-trifluormethyl-2,1,3-benzothiadiazol-5-yl)
N-(4-brom-2,1,3-benzothiadiazol-5-yl)
N-(6-brom-2,1,3-benzothiadiazol-5-yl)
N-(7-brom-2,1,3-benzothiadiazol-5-yl)
N-(4,6-dibrom-2,1,3-benzothiadiazol-5-yl)
N-(4,7-dibrom-2,1,3-benzothiadiazol-5-yl)
N-(6,7-dibrom-2,1,3-benzothiadiazol-5-yl)
N-(4-brom-6-methyl-2,1,3-benzothiadiazol-5-yl)
N-(4-brom-7-methyl-2,1,3-benzothiadiazol-5-yl)
N-(6-brom-7-methyl-2,1,3-benzothiadiazol-5-yl)
N-(4-methyl-6-brom-2,1,3-benzothiadiazol-5-yl)
N-(4-methyl-7-brom-2,1,3-benzothiadiazol-5-yl)
N-(6-methyl-7-brom-2,1,3-benzothiadiazol-5-yl)
N-(4-brom-6-ethyl-2,1,3-benzothiadiazol-5-yl)
N-(4-brom-7-ethyl-2,1,3-benzothiadiazol-5-yl)
N-(6-brom-7-ethyl-2,1,3-benzothiadiazol-5-yl)
N-(4-ethyl-6-brom-2,1,3-benzothiadiazol-5-yl)
N-(4-ethyl-7-brom-2,1,3-benzothiadiazol-5-yl)
N-(6-ethyl-7-brom-2,1,3-benzothiadiazol-5-yl)
N-(4-brom-6-trifluormethyl-2,1,3-benzothiadiazol-5-yl)
N-(4-brom-7-trifluormethyl-2,1,3-benzothiadiazol-5-yl)
N-(6-brom-7-trifluormethyl-2,1,3-benzothiadiazol-5-yl)
N-(4-trifluormethyl-6-brom-2,1,3-benzothiadiazol-5-yl)
N-(4-trifluormethyl-7-brom-2,1,3-benzothiadiazol-5-yl)
N-(6-trifluormethyl-7-brom-2,1,3-benzothiadiazol-5-yl)
N-(4-chlor-2,1,3-benzothiadiazol-5-yl)
N-(6-chlor-2,1,3-benzothiadiazol-5-yl)
N-(7-chlor-2,1,3-benzothiadiazol-5-yl)
N-(4-nitro-2,1,3-benzothiadiazol-5-yl)
N-(6-nitro-2,1,3-benzothiadiazol-5-yl)
N-(7-nitro-2,1,3-benzothiadiazol-5-yl)
N-(4-brom-6-tert.-butyl-2,1,3-benzothiadiazol-5-yl)
N-(4-brom-7-tert.-butyl-2,1,3-benzothiadiazol-5-yl)
N-(6-brom-7-tert.-butyl-2,1,3-benzothiadiazol-5-yl)
N-(4-tert.-butyl-6-brom-2,1,3-benzothiadiazol-5-yl)
N-(4-tert.-butyl-7-brom-2,1,3-benzothiadiazol-5-yl)
N-(6-tert.-butyl-7-brom-2,1,3-benzothiadiazol-5-yl)
N-(4-chlor-6-methyl-2,1,3-benzothiadiazol-5-yl)
N-(4-chlor-7-methyl-2,1,3-benzothiadiazol-5-yl)
N-(6-chlor-7-methyl-2,1,3-benzothiadiazol-5-yl)
N-(4-methyl-6-chlor-2,1,3-benzothiadiazol-5-yl)
N-(4-methyl-7-chlor-2,1,3-benzothiadiazol-5-yl)
N-(6-methyl-7-chlor-2,1,3-benzothiadiazol-5-yl)
N-(4-dimethylamino-2,1,3-benzothiadiazol-5-yl)
N-(6-dimethylamino-2,1,3-benzothiadiazol-5-yl)
N-(7-dimethylamino-2,1,3-benzothiadiazol-5-yl)
N-(4-cyan-2,1,3-benzothiadiazol-5-yl)
N-(6-cyan-2,1,3-benzothiadiazol-5-yl)
N-(7-cyan-2,1,3-benzothiadiazol-5-yl)
N-(4-methoxycarbonyl-2,1,3-benzothiadiazol-5-yl)
N-(6-methoxycarbonyl-2,1,3-benzothiadiazol-5-yl)
N-(7-methoxycarbonyl-2,1,3-benzothiadiazol-5-yl)
N-(4-ethoxycarbonyl-2,1,3-benzothiadiazol-5-yl)
N-(6-ethoxycarbonyl-2,1,3-benzothiadiazol-5-yl)
N-(7-ethoxycarbonyl-2,1,3-benzothiadiazol-5-yl)
N-(4-acetamido-2,1,3-benzothiadiazol-5-yl)
N-(6-acetamido-2,1,3-benzothiadiazol-5-yl)
N-(7-acetamido-2,1,3-benzothiadiazol-5-yl)
bedeutet,
sowie durch analoge Umsetzung mit 2-Ethyl-5-brom-benzolsulfochlorid die nachstehenden 2-Ethyl-5-brom-mZ-1-benzolsulfonamide,
worin mZ
N-(4-methyl-2,1,3-benzothiadiazol-5-yl)
N-(6-methyl-2,1,3-benzothiadiazol-5-yl)
N-(7-methyl-2,1,3-benzothiadiazol-5-yl)
N-(4,6-dimethyl-2,1,3-benzothiadiazol-5-yl)
N-(4,7-dimethyl-2,1,3-benzothiadiazol-5-yl)
N-(6,7-dimethyl-2,1,3-benzothiadiazol-5-yl)
N-(4-trifluormethyl-2,1,3-benzothiadiazol-5-yl)
N-(6-trifluormethyl-2,1,3-benzothiadiazol-5-yl)
N-(7-trifluormethyl-2,1,3-benzothiadiazol-5-yl)
N-(4-brom-2,1,3-benzothiadiazol-5-yl)
N-(6-brom-2,1,3-benzothiadiazol-5-yl)
N-(7-brom-2,1,3-benzothiadiazol-5-yl)
N-(4,6-dibrom-2,1,3-benzothiadiazol-5-yl)
N-(4,7-dibrom-2,1,3-benzothiadiazol-5-yl)
N-(6,7-dibrom-2,1,3-benzothiadiazol-5-yl)
N-(4-brom-6-methyl-2,1,3-benzothiadiazol-5-yl)
N-(4-brom-7-methyl-2,1,3-benzothiadiazol-5-yl)
N-(6-brom-7-methyl-2,1,3-benzothiadiazol-5-yl)
N-(4-methyl-6-brom-2,1,3-benzothiadiazol-5-yl)
N-(4-methyl-7-brom-2,1,3-benzothiadiazol-5-yl)
N-(6-methyl-7-brom-2,1,3-benzothiadiazol-5-yl)
N-(4-brom-6-ethyl-2,1,3-benzothiadiazol-5-yl)
N-(4-brom-7-ethyl-2,1,3-benzothiadiazol-5-yl)
N-(6-brom-7-ethyl-2,1,3-benzothiadiazol-5-yl)
N-(4-ethyl-6-brom-2,1,3-benzothiadiazol-5-yl)
N-(4-ethyl-7-brom-2,1,3-benzothiadiazol-5-yl)
N-(6-ethyl-7-brom-2,1,3-benzothiadiazol-5-yl)
N-(4-brom-6-trifluormethyl-2,1,3-benzothiadiazol-5-yl)
N-(4-brom-7-trifluormethyl-2,1,3-benzothiadiazol-5-yl)
N-(6-brom-7-trifluormethyl-2,1,3-benzothiadiazol-5-yl)
N-(4-trifluormethyl-6-brom-2,1,3-benzothiadiazol-5-yl)
N-(4-trifluormethyl-7-brom-2,1,3-benzothiadiazol-5-yl)
N-(6-trifluormethyl-7-brom-2,1,3-benzothiadiazol-5-yl)
N-(4-chlor-2,1,3-benzothiadiazol-5-yl)
N-(6-chlor-2,1,3-benzothiadiazol-5-yl)
N-(7-chlor-2,1,3-benzothiadiazol-5-yl)
N-(4-nitro-2,1,3-benzothiadiazol-5-yl)
N-(6-nitro-2,1,3-benzothiadiazol-5-yl)
N-(7-nitro-2,1,3-benzothiadiazol-5-yl)
N-(4-brom-6-tert.-butyl-2,1,3-benzothiadiazol-5-yl)
N-(4-brom-7-tert.-butyl-2,1,3-benzothiadiazol-5-yl)
N-(6-brom-7-tert.-butyl-2,1,3-benzothiadiazol-5-yl)
N-(4-tert.-butyl-6-brom-2,1,3-benzothiadiazol-5-yl)
N-(4-tert.-butyl-7-brom-2,1,3-benzothiadiazol-5-yl)
N-(6-tert.-butyl-7-brom-2,1,3-benzothiadiazol-5-yl)
N-(4-chlor-6-methyl-2,1,3-benzothiadiazol-5-yl)
N-(4-chlor-7-methyl-2,1,3-benzothiadiazol-5-yl)
N-(6-chlor-7-methyl-2,1,3-benzothiadiazol-5-yl)
N-(4-methyl-6-chlor-2,1,3-benzothiadiazol-5-yl)
N-(4-methyl-7-chlor-2,1,3-benzothiadiazol-5-yl)
N-(6-methyl-7-chlor-2,1,3-benzothiadiazol-5-yl)
N-(4-dimethylamino-2,1,3-benzothiadiazol-5-yl)
N-(6-dimethylamino-2,1,3-benzothiadiazol-5-yl)
N-(7-dimethylamino-2,1,3-benzothiadiazol-5-yl)
N-(4-cyan-2,1,3-benzothiadiazol-5-yl)
N-(6-cyan-2,1,3-benzothiadiazol-5-yl)
N-(7-cyan-2,1,3-benzothiadiazol-5-yl)
N-(4-methoxycarbonyl-2,1,3-benzothiadiazol-5-yl)
N-(6-methoxycarbonyl-2,1,3-benzothiadiazol-5-yl)
N-(7-methoxycarbonyl-2,1,3-benzothiadiazol-5-yl)
N-(4-ethoxycarbonyl-2,1,3-benzothiadiazol-5-yl)
N-(6-ethoxycarbonyl-2,1,3-benzothiadiazol-5-yl)
N-(7-ethoxycarbonyl-2,1,3-benzothiadiazol-5-yl)
N-(4-acetamido-2,1,3-benzothiadiazol-5-yl)
N-(6-acetamido-2,1,3-benzothiadiazol-5-yl)
N-(7-acetamido-2,1,3-benzothiadiazol-5-yl)
bedeutet,
sowie durch analoge Umsetzung mit 2-Propyl-5-brom-benzolsulfochlorid die nachstehenden 2-Propyl-5-brom-mZ-1-benzolsulfonamide,
worin mZ
N-(4-methyl-2,1,3-benzothiadiazol-5-yl)
N-(6-methyl-2,1,3-benzothiadiazol-5-yl)
N-(7-methyl-2,1,3-benzothiadiazol-5-yl)
N-(4,6-dimethyl-2,1,3-benzothiadiazol-5-yl)
N-(4,7-dimethyl-2,1,3-benzothiadiazol-5-yl)
N-(6,7-dimethyl-2,1,3-benzothiadiazol-5-yl)
N-(4-trifluormethyl-2,1,3-benzothiadiazol-5-yl)
N-(6-trifluormethyl-2,1,3-benzothiadiazol-5-yl)
N-(7-trifluormethyl-2,1,3-benzothiadiazol-5-yl)
N-(4-brom-2,1,3-benzothiadiazol-5-yl)
N-(6-brom-2,1,3-benzothiadiazol-5-yl)
N-(7-brom-2,1,3-benzothiadiazol-5-yl)
N-(4,6-dibrom-2,1,3-benzothiadiazol-5-yl)
N-(4,7-dibrom-2,1,3-benzothiadiazol-5-yl)
N-(6,7-dibrom-2,1,3-benzothiadiazol-5-yl)
N-(4-brom-6-methyl-2,1,3-benzothiadiazol-5-yl)
N-(4-brom-7-methyl-2,1,3-benzothiadiazol-5-yl)
N-(6-brom-7-methyl-2,1,3-benzothiadiazol-5-yl)
N-(4-methyl-6-brom-2,1,3-benzothiadiazol-5-yl)
N-(4-methyl-7-brom-2,1,3-benzothiadiazol-5-yl)
N-(6-methyl-7-brom-2,1,3-benzothiadiazol-5-yl)
N-(4-brom-6-ethyl-2,1,3-benzothiadiazol-5-yl)
N-(4-brom-7-ethyl-2,1,3-benzothiadiazol-5-yl)
N-(6-brom-7-ethyl-2,1,3-benzothiadiazol-5-yl)
N-(4-ethyl-6-brom-2,1,3-benzothiadiazol-5-yl)
N-(4-ethyl-7-brom-2,1,3-benzothiadiazol-5-yl)
N-(6-ethyl-7-brom-2,1,3-benzothiadiazol-5-yl)
N-(4-brom-6-trifluormethyl-2,1,3-benzothiadiazol-5-yl)
N-(4-brom-7-trifluormethyl-2,1,3-benzothiadiazol-5-yl)
N-(6-brom-7-trifluormethyl-2,1,3-benzothiadiazol-5-yl)
N-(4-trifluormethyl-6-brom-2,1,3-benzothiadiazol-5-yl)
N-(4-trifluormethyl-7-brom-2,1,3-benzothiadiazol-5-yl)
N-(6-trifluormethyl-7-brom-2,1,3-benzothiadiazol-5-yl)
N-(4-chlor-2,1,3-benzothiadiazol-5-yl)
N-(6-chlor-2,1,3-benzothiadiazol-5-yl)
N-(7-chlor-2,1,3-benzothiadiazol-5-yl)
N-(4-nitro-2,1,3-benzothiadiazol-5-yl)
N-(6-nitro-2,1,3-benzothiadiazol-5-yl)
N-(7-nitro-2,1,3-benzothiadiazol-5-yl)
N-(4-brom-6-tert.-butyl-2,1,3-benzothiadiazol-5-yl)
N-(4-brom-7-tert.-butyl-2,1,3-benzothiadiazol-5-yl)
N-(6-brom-7-tert.-butyl-2,1,3-benzothiadiazol-5-yl)
N-(4-tert.-butyl-6-brom-2,1,3-benzothiadiazol-5-yl)
N-(4-tert.-butyl-7-brom-2,1,3-benzothiadiazol-5-yl)
N-(6-tert.-butyl-7-brom-2,1,3-benzothiadiazol-5-yl)
N-(4-chlor-6-methyl-2,1,3-benzothiadiazol-5-yl)
N-(4-chlor-7-methyl-2,1,3-benzothiadiazol-5-yl)
N-(6-chlor-7-methyl-2,1,3-benzothiadiazol-5-yl)
N-(4-methyl-6-chlor-2,1,3-benzothiadiazol-5-yl)
N-(4-methyl-7-chlor-2,1,3-benzothiadiazol-5-yl)
N-(6-methyl-7-chlor-2,1,3-benzothiadiazol-5-yl)
N-(4-dimethylamino-2,1,3-benzothiadiazo-5-yl)
N-(6-dimethylamino-2,1,3-benzothiadiazol-5-yl)
N-(7-dimethylamino-2,1,3-benzothiadiazol-5-yl)
N-(4-cyan-2,1,3-benzothiadiazol-5-yl)
N-(6-cyan-2,1,3-benzothiadiazol-5-yl)
N-(7-cyan-2,1,3-benzothiadiazol-5-yl)
N-(4-methoxycarbonyl-2,1,3-benzothiadiazol-5-yl)
N-(6-methoxycarbonyl-2,1,3-benzothiadiazol-5-yl)
N-(7-methoxycarbonyl-2,1,3-benzothiadiazol-5-yl)
N-(4-ethoxycarbonyl-2,1,3-benzothiadiazol-5-yl)
N-(6-ethoxycarbonyl-2,1,3-benzothiadiazol-5-yl)
N-(7-ethoxycarbonyl-2,1,3-benzothiadiazol-5-yl)
N-(4-acetamido-2,1,3-benzothiadiazol-5-yl)
N-(6-acetamido-2,1,3-benzothiadiazol-5-yl)
N-(7-acetamido-2,1,3-benzothiadiazol-5-yl)
bedeutet.

### Beispiel 6

Analog Beispiel 1 erhält man durch Umsetzung von 6-Amino-1,2,5-oxadiazolo-[3,4-b]-pyridin mit "A" das 5-Dimethylamino-N-(1,2,5-oxadiazolo-[3,4-b]-pyridin-6-yl)-1-naphthalinsulfonamid.

Analog erhält man aus folgenden mZ-6-Amino-1,2,5-oxadiazolo-[3,4-b]-pyridinen,
worin mZ
5-Methyl
7-Methyl
5,7-Dimethyl
5-Trifluormethyl
7-Trifluormethyl
5-Brom
7-Brom
5,7-Dibrom
5-Brom-7-methyl
5-Methyl-7-brom
5-Brom-7-ethyl
5-Ethyl-7-brom
5-Brom-7-trifluormethyl
5-Trifluormethyl-7-brom
5-Chlor
7-Chlor
5-Nitro
7-Nitro
5-Brom-7-tert.-butyl
5-tert.-Butyl-7-brom
5-Chlor-7-methyl
5-Methyl-7-chlor
5-Dimethylamino
7-Dimethylamino
5-Cyan
7-Cyan
5-Methoxycarbonyl
7-Methoxycarbonyl
5-Ethoxycarbonyl
7-Ethoxycarbonyl
5-Acetamino
7-Acetamid
bedeutet
durch Umsetzung mit "A" die nachstehenden 5-Dimethylamino-N-(mZ-1,2,5-oxadiazolo-[3,4-b]-pyridin-6-yl)-1-naphthalinsulfonamide,
worin mZ
5-Methyl
7-Methyl
5,7-Dimethyl
5-Trifluormethyl
7-Trifluormethyl
5-Brom
7-Brom
5,7-Dibrom
5-Brom-7-methyl
5-Methyl-7-brom
5-Brom-7-ethyl
5-Ethyl-7-brom
5-Brom-7-trifluormethyl
5-Trifluormethyl-7-brom
5-Chlor
7-Chlor
5-Nitro
7-Nitro
5-Brom-7-tert.-butyl
5-tert.-Butyl-7-brom
5-Chlor-7-methyl
5-Methyl-7-chlor
5-Dimethylamino
7-Dimethylamino
5-Cyan
7-Cyan
5-Methoxycarbonyl
7-Methoxycarbonyl
5-Ethoxycarbonyl
7-Ethoxycarbonyl
5-Acetamido
7-Acetamido
bedeutet.

Analog erhält man durch Umsetzung von 6-Amino-1,2,5-thiadiazolo-[3,4-b]-pyridin mit "A" das 5-Dimethylamino-N-(1,2,5-thiadiazolo-[3,4-b]-pyridin-6-yl)-1-naphthalinsulfonamid.

Analog erhält man aus folgenden mZ-6-Amino-1,2,5-thiadiazolo-[3,4-b]-pyridinen,
worin mZ
5-Methyl
7-Methyl
5,7-Dimethyl
5-Trifluormethyl
7-Trifluormethyl
5-Brom
7-Brom
5,7-Dibrom
5-Brom-7-methyl
5-Methyl-7-brom
5-Brom-7-ethyl
5-Ethyl-7-brom
5-Brom-7-trifluormethyl
5-Trifluormethyl-7-brom
5-Chlor
7-Chlor
5-Nitro
7-Nitro
5-Brom-7-tert.-butyl
5-tert.-Butyl-7-brom
5-Chlor-7-methyl
5-Methyl-7-chlor
5-Dimethylamino
7-Dimethylamino
5-Cyan
7-Cyan
5-Methoxycarbonyl
7-Methoxycarbonyl
5-Ethoxycarbonyl
7-Ethoxycarbonyl
5-Acetamido
7-Acetamido
bedeutet
durch Umsetzung mit "A" die nachstehenden 5-Dimethylamino-N-(mZ-1,2,5-thiadiazolo-[3,4-b]-pyridin-6-yl)-1-naphthalinsulfonamide,
worin mZ
5-Methyl, F. 192°
7-Methyl
5,7-Dimethyl
5-Trifluormethyl
7-Trifluormethyl
5-Brom
7-Brom
5,7-Dibrom
5-Brom-7-methyl
5-Methyl-7-brom
5-Brom-7-ethyl
5-Ethyl-7-brom
5-Brom-7-trifluormethyl
5-Trifluormethyl-7-brom
5-Chlor
7-Chlor
5-Nitro
7-Nitro
5-Brom-7-tert.-butyl
5-tert.-Butyl-7-brom
5-Chlor-7-methyl
5-Methyl-7-chlor
5-Dimethylamino
7-Dimethylamino
5-Cyan
7-Cyan
5-Methoxycarbonyl
7-Methoxycarbonyl
5-Ethoxycarbonyl
7-Ethoxycarbonyl
5-Acetamido
7-Acetamido
bedeutet.

Analog erhält man durch Umsetzung von 5-Amino-1,2,5-thiadiazolo-[3,4-b]pyridin mit "A" das 5-Dimethylamin-N-(1,2,5-thiadiazolo-[3,4-b]-5-yl)-1-naphthalinsulfonamid.

Analog erhält man durch Umsetzung von 7-Amino-1,2,5-oxadiazolo-[3,4-b]-pyridin mit "A" das 5-Dimethylamino-N-(1,2,5-oxadiazolo-[3,4-b]-pyridin-7-yl)-1-naphthalinsulfonamid.

Analog erhält man aus folgenden mZ-7-Amino-1,2,5-oxadiazolo-[3,4-b]-pyridinen,
worin mZ
5-Methyl
6-Methyl
5,6-Dimethyl
5-Trifluormethyl
6-Trifluormethyl
5-Brom
6-Brom
5,6-Dibrom
5-Brom-6-methyl
5-Methyl-6-brom
5-Brom-6-ethyl
5-Ethyl-6-brom
5-Brom-6-trifluormethyl
5-Trifluormethyl-6-brom
5-Chlor
6-Chlor
5-Nitro
6-Nitro
5-Brom-6-tert.-butyl
5-tert.-Butyl-6-brom
5-Chlor-6-methyl
5-Methyl-6-chlor
5-Dimethylamino
6-Dimethylamino
5-Cyan
6-Cyan
5-Methoxycarbonyl
6-Methoxycarbonyl
5-Ethoxycarbonyl
6-Ethoxycarbonyl
5-Acetamido
6-Acetamido
bedeutet
durch Umsetzung mit "A" die nachstehenden 5-Dimethylamino-N-(mZ-1,2,5-oxadiazolo-[3,4-b]-pyridin-6-yl)-1-naphthalinsulfonamide,
worin mZ
5-Methyl
6-Methyl
5,6-Dimethyl
5-Trifluormethyl
6-Trifluormethyl
5-Brom
6-Brom
5,6-Dibrom
5-Brom-6-methyl
5-Methyl-6-brom
5-Brom-6-ethyl
5-Ethyl-6-brom
5-Brom-6-trifluormethyl
5-Trifluormethyl-6-brom
5-Chlor
6-Chlor
5-Nitro
6-Nitro
5-Brom-6-tert.-butyl
5-tert.-Butyl-6-brom
5-Chlor-6-methyl
5-Methyl-6-chlor
5-Dimethylamino
6-Dimethylamino
5-Cyan
6-Cyan
5-Methoxycarbonyl
6-Methoxycarbonyl
5-Ethoxycarbonyl
6-Ethoxycarbonyl
5-Acetamido
6-Acetamido
bedeutet.

Analog erhält man durch Umsetzung von 7-Amino-1,2,5-thiadiazolo-[3,4-b]-pyridin mit "A" das 5-Dimethylamino-N-(1,2,5-thiadiazolo-[3,4-b]-pyridin-7-yl)-1-naphthalinsulfonamid.

Analog erhält man aus folgenden mZ-7-Amino-1,2,5-thiadiazolo-[3,4-b]-pyridinen,
worin mZ
5-Methyl
6-Methyl
5,6-Dimethyl
5-Trifluormethyl
6-Trifluormethyl
5-Brom
6-Brom
5,6-Dibrom
5-Brom-6-methyl
5-Methyl-6-brom
5-Brom-6-ethyl
5-Ethyl-6-brom
5-Brom-6-trifluormethyl
5-Trifluormethyl-6-brom
5-Chlor
6-Chlor
5-Nitro
6-Nitro
5-Brom-6-tert.-butyl
5-tert.-Butyl-6-brom
5-Chlor-6-methyl
5-Methyl-6-chlor
5-Dimethylamino
6-Dimethylamino
5-Cyan
6-Cyan
5-Methoxycarbonyl
6-Methoxycarbonyl
5-Ethoxycarbonyl
6-Ethoxycarbonyl
5-Acetamido
6-Acetamido
bedeutet
durch Umsetzung mit "A" die nachstehenden 5-Dimethylamino-N-(mZ-1,2,5-thiadiazolo-[3,4-b]-pyridin-6-yl)-1-naphthalinsulfonamide,
worin mZ
5-Methyl
6-Methyl
5,6-Dimethyl
5-Trifluormethyl
6-Trifluormethyl
5-Brom
6-Brom
5,6-Dibrom
5-Brom-6-methyl
5-Methyl-6-brom
5-Brom-6-ethyl
5-Ethyl-6-brom
5-Brom-6-trifluormethyl
5-Trifluormethyl-6-brom
5-Chlor
6-Chlor
5-Nitro
6-Nitro
5-Brom-6-tert.-butyl
5-tert.-Butyl-6-brom
5-Chlor-6-methyl
5-Methyl-6-chlor
5-Dimethylamino
6-Dimethylamino
5-Cyan
6-Cyan
5-Methoxycarbonyl
6-Methoxycarbonyl
5-Ethoxycarbonyl
6-Ethoxycarbonyl
5-Acetamido
6-Acetamido
bedeutet.

### Beispiel 7

Analog Beispiel 1 erhält man durch Umsetzung von 4-Amino-2,1,3-benzoxadiazol mit "A" 5-Dimethylamino-N-(2,1,3-benzoxadiazol-4-yl)-1-naphthalinsulfonamid,
und aus den folgenden 4-Amino-mZ-2,1,3-benzoxadiazolen, worin mZ
5-Methyl
6-Methyl
7-Methyl
5,6-Dimethyl
5,7-Dimethyl
6,7-Dimethyl
5-Trifluormethyl
6-Trifluormethyl
7-Trifluormethyl
5-Brom
6-Brom
7-Brom
5,6-Dibrom
5,7-Dibrom
6,7-Dibrom
5-Brom-6-methyl
5-Brom-7-methyl
6-Brom-7-methyl
5-Methyl-6-brom
5-Methyl-7-brom
6-Methyl-7-brom
5-Brom-6-ethyl
5-Brom-7-ethyl
6-Brom-7-ethyl
5-Ethyl-6-brom
5-Ethyl-7-brom
6-Ethyl-7-brom
5-Brom-6-trifluormethyl
5-Brom-7-trifluormethyl
6-Brom-7-trifluormethyl
5-Trifluormethyl-6-brom
5-Trifluormethyl-7-brom
6-Trifluormethyl-7-brom
5-Chlor
6-Chlor
7-Chlor
5-Nitro
6-Nitro
7-Nitro-
5-Brom-6-tert.-butyl
5-Brom-7-tert.-butyl
6-Brom-7-tert.-butyl
5-tert.-Butyl-6-brom
5-tert.-Butyl-7-brom
6-tert.-Butyl-7-brom
5-Chlor-6-methyl
5-Chlor-7-methyl
6-Chlor-7-methyl
5-Methyl-6-chlor
5-Methyl-7-chlor
6-Methyl-7-chlor
5-Dimethylamino
6-Dimethylamino
7-Dimethylamino
5-Cyan
6-Cyan
7-Cyan
5-Methoxycarbonyl
6-Methoxycarbonyl
7-Methoxycarbonyl
5-Ethoxycarbonyl
6-Ethoxycarbonyl
7-Ethoxycarbonyl
5-Acetamido
6-Acetamido
7-Acetamido
bedeutet
mit "A" die nachstehenden 5-Dimethylamino-mZ-1-naphthalinsulfonamide, worin mZ
N-(5-methyl-2,1,3-benzoxadiazol-4-yl)
N-(6-methyl-2,1,3-benzoxadiazol-4-yl)
N-(7-methyl-2,1,3-benzoxadiazol-4-yl)
N-(5,6-dimethyl-2,1,3-benzoxadiazol-4-yl)
N-(5,7-dimethyl-2,1,3-benzoxadiazol-4-yl)
N-(6,7-dimethyl-2,1,3-benzoxadiazol-4-yl)
N-(5-trifluormethyl-2,1,3-benzoxadiazol-4-yl)
N-(6-trifluormethyl-2,1,3-benzoxadiazol-4-yl)
N-(7-trifluormethyl-2,1,3-benzoxadiazol-4-yl)
N-(5-brom-2,1,3-benzoxadiazol-4-yl)
N-(6-brom-2,1,3-benzoxadiazol-4-yl)
N-(7-brom-2,1,3-benzoxadiazol-4-yl)
N-(5,6-dibrom-2,1,3-benzoxadiazol-4-yl)
N-(5,7-dibrom-2,1,3-benzoxadiazol-4-yl)
N-(6,7-dibrom-2,1,3-benzoxadiazol-4-yl)
N-(5-brom-6-methyl-2,1,3-benzoxadiazol-4-yl)
N-(5-brom-7-methyl-2,1,3-benzoxadiazol-4-yl)
N-(6-brom-7-methyl-2,1,3-benzoxadiazol-4-yl)
N-(5-methyl-6-brom-2,1,3-benzoxadiazol-4-yl)
N-(5-methyl-7-brom-2,1,3-benzoxadiazol-4-yl)
N-(6-methyl-7-brom-2,1,3-benzoxadiazol-4-yl)
N-(5-brom-6-ethyl-2,1,3-benzoxadiazol-4-yl)
N-(5-brom-7-ethyl-2,1,3-benzoxadiazol-4-yl)
N-(6-brom-7-ethyl-2,1,3-benzoxadiazol-4-yl)
N-(5-ethyl-6-brom-2,1,3-benzoxadiazol-4-yl)
N-(5-ethyl-7-brom-2,1,3-benzoxadiazol-4-yl)
N-(6-ethyl-7-brom-2,1,3-benzoxadiazol-4-yl)
N-(5-brom-6-trifluormethyl-2,1,3-benzoxadiazol-4-yl)
N-(5-brom-7-trifluormethyl-2,1,3-benzoxadiazol-4-yl)
N-(6-brom-7-trifluormethyl-2,1,3-benzoxadiazol-4-yl)
N-(5-trifluormethyl-6-brom-2,1,3-benzoxadiazol-4-yl)
N-(5-trifluormethyl-7-brom-2,1,3-benzoxadiazol-4-yl)
N-(6-trifluormethyl-7-brom-2,1,3-benzoxadiazol-4-yl)
N-(5-chlor-2,1,3-benzoxadiazol-4-yl)
N-(6-chlor-2,1,3-benzoxadiazol-4-yl)
N-(7-chlor-2,1,3-benzoxadiazol-4-yl)
N-(5-nitro-2,1,3-benzoxadiazol-4-yl)
N-(6-nitro-2,1,3-benzoxadiazol-4-yl)
N-(7-nitro-2,1,3-benzoxadiazol-4-yl)
N-(5-brom-6-tert.-butyl-2,1,3-benzoxadiazol-4-yl)
N-(5-brom-7-tert.-butyl-2,1,3-benzoxadiazol-4-yl)
N-(6-brom-7-tert.-butyl-2,1,3-benzoxadiazol-4-yl)
N-(5-tert.-butyl-6-brom-2,1,3-benzoxadiazol-4-yl)
N-(5-tert.-butyl-7-brom-2,1,3-benzoxadiazol-4-yl)
N-(6-tert.-butyl-7-brom-2,1,3-benzoxadiazol-4-yl)
N-(5-chlor-6-methyl-2,1,3-benzoxadiazol-4-yl)
N-(5-chlor-7-methyl-2,1,3-benzoxadiazol-4-yl)
N-(6-chlor-7-methyl-2,1,3-benzoxadiazol-4-yl)
N-(5-methyl-6-chlor-2,1,3-benzoxadiazol-4-yl)
N-(5-methyl-7-chlor-2,1,3-benzoxadiazol-4-yl)
N-(6-methyl-7-chlor-2,1,3-benzoxadiazol-4-yl)
N-(5-dimethylamino-2,1,3-benzoxadiazol-4-yl)
N-(6-dimethylamino-2,1,3-benzoxadiazol-4-yl)
N-(7-dimethylamino-2,1,3-benzoxadiazol-4-yl)
N-(5-cyan-2,1,3-benzoxadiazol-4-yl)
N-(6-cyan-2,1,3-benzoxadiazol-4-yl)
N-(7-cyan-2,1,3-benzoxadiazol-4-yl)
N-(5-methoxycarbonyl-2,1,3-benzoxadiazol-4-yl)
N-(6-methoxycarbonyl-2,1,3-benzoxadiazol-4-yl)
N-(7-methoxycarbonyl-2,1,3-benzoxadiazol-4-yl)
N-(5-ethoxycarbonyl-2,1,3-benzoxadiazol-4-yl)
N-(6-ethoxycarbonyl-2,1,3-benzoxadiazol-4-yl)
N-(7-ethoxycarbonyl-2,1,3-benzoxadiazol-4-yl)
N-(5-acetamido-2,1,3-benzoxadiazol-4-yl)
N-(6-acetamido-2,1,3-benzoxadiazol-4-yl)
N-(7-acetamido-2,1,3-benzoxadiazol-4-yl)
bedeutet.

### Beispiel 8

Ein Gemisch von 4,6 g 5-Dimethylamino-N-(5-brom-6-ethyl-2,1,3-benzoxadiazol-4-yl)-1-naphthalinsulfonamid und 1,3 g Kupfercyanid in 30 ml Pyridin wird 8 Stunden bei 120° erhitzt. Man gießt auf wässrige Ammoniaklösung, arbeitet wie üblich auf und erhält 5-Dimethylamino-N-(5-cyan-6-ethyl-2,1,3-benzoxadiazol-4-yl)-1-naphthalinsulfonamid.

### Beispiel 9

Eine Lösung von 1 g 5-Dimethylamino-N-(5-cyan-6-ethyl-2,1,3-benzoxadiazol-4-yl)-1-naphthalinsulfonamd und 0,7 g Kaliumhydroxid in 20 ml Ethanol und 5 ml Wasser wird 8 Stunden unter Rühren gekocht. Man entfernt die Lösungsmittel, löst in Wasser und versetzt mit Salzsäure und erhält 5-Dimethylamino-N-(5-carboxy-6-ethyl-2,1,3-benzoxadiazol-4-yl)-1-naphthalinsulfonamid.

### Beispiel 10

Eine Lösung von 1 g 5-Dimethylamino-N-(5-carboxy-6-ethyl-2,1,3-benzoxadiazol-4-yl)-1-naphthalinsulfonamid, 0,5 ml konz. Schwefelsäure und 30 ml Ethanol wird 6 Stunden bei 80° erhitzt. Man entfernt das Lösungsmittel, arbeitet wie üblich auf und erhält 5-Dimethylamino-N-(5-ethoxycarbonyl-6-ethyl-2,1,3-benzoxadiazol-4-yl)-1-naphthalinsulfonamid.

### Beispiel 11

Eine Lösung von 6 g 4-Amino-N-(2,1,3-benzothiadiazol-5-yl)-1-benzolsulfonamid und 0,5 g Titantetrachlorid in 100 ml Methanol wird mit 1 ml frisch destilliertem Acetaldehyd versetzt. Anschließend gibt man 4 g Natriumcyanborhydrid dazu und rührt 30 Stunden. Man gibt kalte halbkonzentrierte Salzsäure dazu, arbeitet wie üblich auf und erhält 4-Ethylamino-N-(2,1,3-benzothiadiazol-5-yl)-1-benzolsulfonamid.

### Beispiel 12

Eine Lösung von 1 g 4-Isocyanato-N-(4-brom-7-methyl-2,1,3-benzothiadiazol-5-yl)-1-benzolsulfonamid [erhältlich durch Umsetzung von 4-Brom-5-amino-7-methyl-2,1,3-benzothiadiazol mit 4-Isocyanatobenzosulfochlorid] in 80 ml Toluol wird mit 0,5 g Pyrrolidin versetzt und 1 Stunde bei 90° gerührt. Nach üblicher Aufarbeitung erhält man 4-Pyrrolidino-amido-N-(4-brom-7-methyl-2,1,3-benzothiadiazol-5-yl)-1-benzolsulfonamid.

### Beispiel 13

Analog Beispiel 1 erhält man durch Umsetzung von 5-Amino-2,1,3-benzothiadiazol mit den nachstehnden M-naphthalin-sulfochloriden, worin M
5-Diethylamino
5-Isopropylamino
5-Isopropyl-methyl-amino
5-Methylamino
5-Ethylamino
5-Propylamino
5-Butylamino
5-Pentylamino
bedeutet,
die folgenden M-N-(2,1,3-benzothiadiazol-5-yl)-1-naphthalinsulfonamide,
worin M
5-Diethylamino
5-Isopropylamino
5-Isopropyl-methyl-amino
5-Methylamino
5-Ethylamino
5-Propylamino
5-Butylamino
5-Pentylamino
bedeutet.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 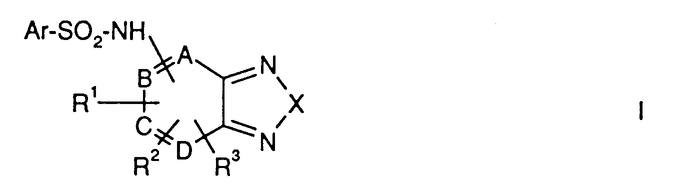 worin
-A=B-C=D- eine -CH=CH-CH=CH-Gruppe,in der auch 1 oder 2 CH durch N ersetzt sein können,
Ar unsubstituiertes oder ein-, zwei- oder dreifach durch H, Hal, Q, Alkenyl mit bis zu 6 C-Atomen, Ph, OPh, NO₂, NR⁴R⁵, NHCOR⁴, CF₃, OCF₃, CN, OR⁴, COOR⁴, (CH₂)ₙCOOR⁴, (CH₂)ₙNR⁴R⁵, -N=C=O oder NHCONR⁴R⁵ substituiertes Ph oder Naphthyl,
R¹, R², R³ jeweils unabhängig voneinander fehlen, H, Hal, Q, CF₃, NO₂, NR⁴R⁵, CN, COOR⁴ oder NHCOR⁴,
R⁴, R⁵ jeweils unabhängig voneinander H oder Q, oder zusammen auch -CH₂-(CH₂)ₙ-CH₂-,
Q Alkyl mit 1 bis 6 C-Atomen,
Ph Phenyl,
X O oder S,
Hal F, Cl, Br oder I,
n 1, 2 oder 3 bedeuten,
sowie ihre Salze,
ausgenommen
4-Methyl-N-(2,1,3-benzothiadiazol-4-yl)-benzolsulfonamid, 4-Methyl-N-(2,1,3-benzothiadiazol-5-yl)-benzolsulfonamid, 4-Nitro-N-(2,1,3-benzothiadiazol-4-yl)-benzolsulfonamid, 4-Nitro-N-(2,1,3-benzothiadiazol-5-yl)-benzolsulfonamid, 4-Amino-N-(2,1,3-benzothiadiazol-4-yl)-benzolsulfonamid und 4-Amino-N-(2,1,3-benzothiadiazol-5-yl)-benzolsulfonamid.

2. a) 5-Brom-2-ethyl-N-(2,1,3-benzothiadiazol-5-yl)-benzolsulfonamid;
b) 2,5-Dichlor-N-(2,1,3-benzothiadiazol-5-yl)-benzolsulfonamid;
c) 5-Brom-2-propyl-N-(2,1,3-benzothiadiazol-5-yl)-benzolsulfonamid;
d) 5-Dimethylamino-N-(2,1,3-benzothiadiazol-5-yl)-naphthalinsulfonamid;
e) 5-Dimethylamino-N-[6-methyl-(2,1,3-benzothiadiazol-5-yl)]-naphthalinsulfonamid;
f) 5-Dimethylamino-N-[4-brom-(2,1,3-benzothiadiazol-5-yl)]-naphthalinsulfonamid;
g) 5-Dimethylamino-N-(2,1,3-benzothiadiazol-4-yl)-naphthalinsulfonamid;
h) 5-Dimethylamino-N-([1,2,5]-oxadiazole-[3,4-b]-pyridin-6-yl)-naphthalinsulfonamid; i) 5-Dimethylamino-N-(1,2,5-benzoxadiazol-5-yl)-1-naphthalin
sulfonamid;
j) 5-Dimethylamino-N-(6-Brom-7-methyl-1,2,5-benzoxadiazol-5-yl)-1-naphthalinsulfonamid;
k) 2-Phenyl-N-(2,1,3-benzothiadiazol-5-yl)-benzolsulfonamid;
nach Anspruch 1.

3. Verfahren zur Herstellung von Verbindungen der Formel I nach Anspruch 1 sowie ihrer Salze, dadurch gekennzeichnet, daß man
(a) eine Verbindung der Formel II 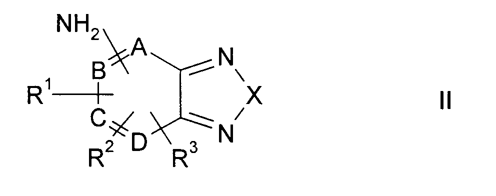 worin
-A=B-C=D-, R¹, R², R³ und X die in Anspruch 1 angegebenen Bedeutungen haben,
mit einer Verbindung der Formel III
Ar-SO₂-E III
worin
E Cl, Br, I oder eine freie oder reaktionsfähig funktionell abgewandelte OH-Gruppe bedeutet und
Ar die in Anspruch 1 angegebene Bedeutung hat,
oder
b) daß man zur Herstellung einer Verbindung der Formel I,
worin
X S bedeutet
eine Verbindung der Formel IV 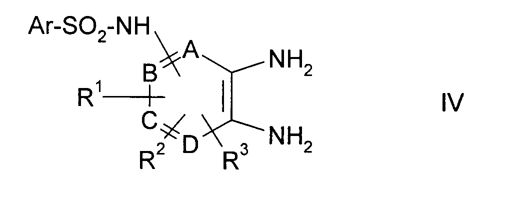
worin
-A=B-C=D-, Ar, R¹, R² und R³ die in Anspruch 1 angegebenen Bedeutungen haben,
mit Thionylchlorid oder einem reaktionsfähigen Derivat dieser Verbindung
umsetzt,
oder
c) daß man zur Herstellung einer Verbindung der Formel I,
worin
X O bedeutet,
eine Verbindung der Formel V 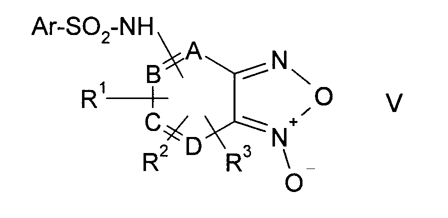
worin
-A=B-C=D-, Ar, R¹, R² und R³ die in Anspruch 1 angegebenen Bedeutungen haben,
reduziert,
und/oder daß man in einer Verbindung der Formel I einen oder mehrere Rest(e) R¹, R² und/oder R³ in einen oder mehrere Reste R¹, R² und/oder R³ umwandelt,
indem man
i) eine Nitrogruppe zu einer Aminogruppe reduziert,
ii) einen Bromsubstituenten durch eine Cyangruppe ersetzt,
iii) eine Cyangruppe zu einer Carboxygruppe hydrolysiert,
iv) eine Carboxygruppe verestert,
v) eine Aminogruppe durch reduktive Aminierung in ein alkyliertes Amin umwandelt
und/oder eine Base oder Säure der Formel I in eines ihrer Salze umwandelt.

4. Verfahren zur Herstellung pharmazeutischer Zubereitungen, dadurch gekennzeichnet, daß man eine Verbindung der Formel I nach Anspruch 1 und/oder 4-Methyl-N-(2,1,3-benzothiadiazol-5-yl)-benzolsulfonamid und/oder 4-Nitro-N-(2,1,3-benzothiadiazol-5-yl)-benzolsulfonamid und/oder 4-Amino-N-(2,1,3-benzothiadiazol-5-yl)-benzolsulfonamid und/oder eines ihrer physiologischen unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder 4-Methyl-N-(2,1,3-benzothiadiazol-5-yl)-benzolsulfonamid und/oder 4-Nitro-N-(2,1,3-benzothiadiazol-5-yl)-benzolsulfonamid und/oder 4-Amino-N-(2,1,3-benzothiadiazol-5-yl)-benzolsulfonamid und/oder einem ihrer physiologisch unbedenklichen Salze.

6. Verbindungen der Formel I nach Anspruch 1 und 4-Methyl-N-(2,1,3-benzothiadiazol-5-yl)-benzolsulfonamid und 4-Nitro-N-(2,1,3-benzothiadiazol-5-yl)-benzolsulfonamid und 4-Amino-N-(2,1,3-benzothiadiazol-5-yl)-benzolsulfonamid und ihre physiologisch unbedenklichen Salze zur Bekämpfung von Krankheiten.

7. Arzneimittel der Formel I nach Anspruch 1 und 4-Methyl-N-(2,1,3-benzothiadiazol-5-yl)-benzolsulfonamid und 4-Nitro-N-(2,1,3-benzothiadiazol-5-yl)-benzolsulfonamid und 4-Amino-N-(2,1,3-benzothiadiazol-5-yl)-benzolsulfonamid und ihre physiologisch unbedenklichen Salze als Endothelin-Rezeptor-Antagonisten.

8. Verwendung von Verbindungen der Formel I nach Anspruch 1 und/oder 4-Methyl-N-(2,1,3-benzothiadiazol-5-yl)-benzolsulfonamid und/oder 4-Nitro-N-(2,1,3-benzothiadiazol-5-yl)-benzolsulfonamid und/oder 4-Amino-N-(2,1,3-benzothiadiazol-5-yl)-benzolsulfonamid und/oder ihre physiologisch unbedenklichen Salze zur Herstellung eines Arzneimittels.

## Claims

1. Compounds of the formula I 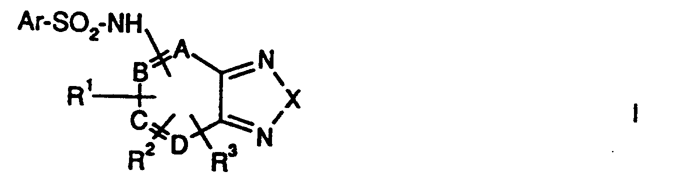
in which
-A=B-C=D- is a -CH=CH-CH=CH- group in which 1 or 2 CH groups can also be replaced by N,
Ar is Ph or naphthyl, which is unsubstituted or mono-, di- or trisubstituted by H, Hal, Q, alkenyl having up to 6 C atoms, Ph, OPh, NO₂, NR⁴R⁵, NHCOR⁴, CF₃, OCF₃, CN, OR⁴, COOR⁴, (CH₂)ₙCOOR⁴, (CH₂)ₙNR⁴R⁵, -N=C=O or NHCONR⁴R⁵,
R¹, R² and R³ in each case independently of one another are absent, H, Hal, Q, CF₃, NO₂, NR⁴R⁵, CN, COOR⁴ or NHCOR⁴,
R⁴ and R⁵ in each case independently of one another are H or Q, or together are also -CH₂-(CH₂)ₙ-CH₂₋,
Q is alkyl having 1 to 6 C atoms,
Ph is phenyl,
X is O or S,
Hal is F, Cl, Br or I,
n is 1, 2 or 3,
and also their salts,
excluding
4-methyl-N-(2,1,3-benzothiadiazol-4-yl)benzenesulfonamide, 4-methyl-N-(2,1,3-benzothiadiazol-5-yl)benzenesulfonamide, 4-nitro-N-(2,1,3-benzothiadiazol-4-yl)benzenesulfonamide, 4-nitro-N-(2,1,3-benzothiadiazol-5-yl)benzenesulfonamide, 4-amino-N-(2,1,3-benzothiadiazol-4-yl)benzenesulfonamide and 4-amino-N-(2,1,3-benzothiadiazol-5-yl)benzenesulfonamide.

2. a) 5-Bromo-2-ethyl-N-(2,1,3-benzothiadiazol-5-yl)benzene sulfonamide;
b) 2,5-dichloro-N-(2,1,3-benzothiadiazol-5-yl)benzene sulfonamide;
c) 5-bromo-2-propyl-N-(2,1,3-benzothiadiazol-5-yl)benzene sulfonamid;
d) 5-dimethylamino-N-(2,1,3-benzothiadiazol-5-yl)naphthalenesulfonamide;
e) 5-dimethylamino-N-[6-methyl-(2,1,3-benzothiadiazol-5-yl)]naphthalenesulfonamide;
f) 5-dimethylamino-N-[4-bromo-(2,1,3-benzothiadiazol-5-yl)]naphthalenesulfonamide;
g) 5-dimethylamino-N-(2,1,3-benzothiadiazol-4-yl)naphthalenesulfonamide;
h) 5-dimethylamino-N-(1,2,5oxadiazole-[3,4-b]pyridin-6-yl)naphthalenesulfonamide;
i) 5-dimethylamino-N-(1,2,5-benzoxadiazol-5-yl)-1-naphthalenesulfonamide;
j) 5-dimethylamino-N-(6-bromo-7-methyl-1,2,5-benzoxadiazol-5-yl)-1-naphthalenesulfonamide;
k) 2-phenyl-N-(2,1,3-benzothiadiazol-5-yl)benzenesulfonamide; according to Claim 1.

3. Process for the preparation of compounds of the formula I according to Claim 1 and their salts, characterized in that
(a) a compound of the formula II 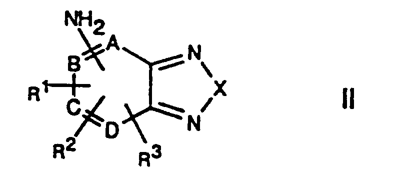
in which
-A=B-C=D-, R¹, R², R³ and X have the meanings indicated in Claim 1,
is reacted with a compound of the formula III
Ar-SO₂-E III
in which
E is Cl, Br, I or a free or reactive functionally modified OH group and
Ar has the meaning indicated in Claim 1,
or
(b) in that for the preparation of a compound of the formula I,
in which
X is S,
a compound of the formula IV 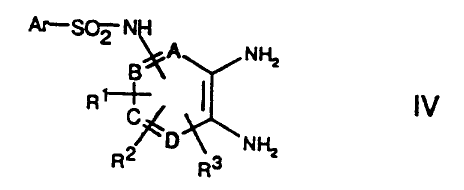
in which
-A=B-C=D-, Ar, R¹, R² and R³ have the meanings indicated in Claim 1,
is reacted with thionyl chloride or a reactive derivative of this compound,
or
c) in that for the preparation of a compound of the formula I
in which
X is O
a compound of the formula V 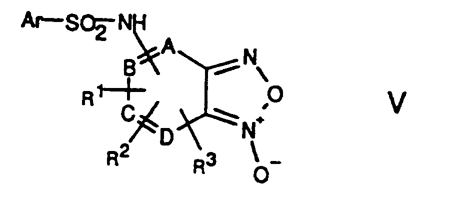
in which
-A=B-C=D-, Ar, R¹, R² and R³ have the meanings indicated in Claim 1,
is reduced,
and/or in that in a compound of the formula I one or more radicals R¹, R² and/or R³ is converted into one or more other radicals R¹, R² and/or R³,
by
i) reducing a nitro group to an amino group,
ii) replacing a bromo substituent by a cyano group,
iii) hydrolysing a cyano group to a carboxyl group,
iv) esterifying a carboxyl group,
v) converting an amino group by reductive amination into an alkylated amine
and/or converting a base or acid of the formula I into one of its salts.

4. Process for the production of pharmaceutical preparations, characterized in that a compound of the formula I according to Claim 1 and/or 4-methyl-N-(2,1,3-benzothiadiazol-5-yl)benzenesulfonamide and/or 4-nitro-N-(2,1,3-benzothiadiazol-5-yl)benzenesulfonamide and/or 4-amino-N-(2,1,3-benzothiadiazol-5-yl)-benzenesulfonamide and/or one of its physiologically acceptable salts is brought into a suitable dose form together with at least one solid, liquid or semi-liquid excipient or auxiliary.

5. Pharmaceutical preparation, characterized in that it contains at least one compound of the formula I according to Claim 1 and/or 4-methyl-N-(2,1,3-benzothiadiazol-5-yl)benzenesulfonamide and/or 4-nitro-N-(2,1,3-benzothiadiazol-5-yl)benzenesulfonamide and/or 4-amino-N-(2,1,3-benzothiadiazol-5-yl)benzenesulfonamide and/or one of their physiologically acceptable salts.

6. Compounds of the formula I according to Claim 1 and 4-methyl-N-(2,1,3-benzothiadiazol-5-yl)benzenesulfonamide and 4-nitro-N-(2,1,3-benzothiadiazol-5-yl)benzenesulfonamide and 4-amino-N-(2,1,3-benzothiadiazol-5-yl)benzenesulfonamide and their physiologically acceptable salts for the control of disorders.

7. Medicament of the formula I according to Claim 1 and 4-methyl-N-(2,1,3-benzothiadiazol-5-yl)benzenesulfonamide and 4-nitro-N-(2,1,3-benzothiadiazol-5-yl)benzenesulfonamide and 4-amino-N-(2,1,3-benzothiadiazol-5-yl)benzenesulfonamide and their physiologically acceptable salts as endothelin receptor antagonists.

8. Use of compounds of the formula I according to Claim 1 and/or 4-methyl-N-(2,1,3-benzothiadiazol-5-yl)benzenesulfonamide and/or 4-nitro-N-(2,1,3-benzothiadiazol-5-yl)benzenesulfonamide and/or 4-amino-N-(2,1,3-benzothiadiazol-5-yl)benzenesulfonamide and/or their physiologically acceptable salts for the production of a medicament.

## Revendications

1. Composés de formule I 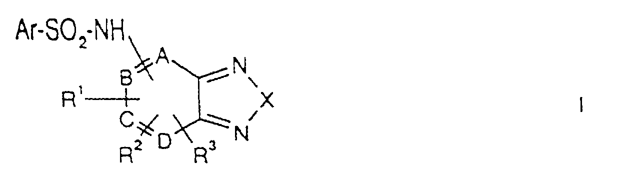
dans laquelle
-A=B-C=D- représente un groupe -CH=CH-CH=CH-, dans lequel un ou deux CH peuvent également être remplacés par N,
Ar représente un phényle ou un naphtyle non substitué ou mono-, bi- ou trisubstitué par H, Hal, Q, un alcényle ayant jusqu'à 6 atomes de carbone, Ph, OPh, NO₂, NR⁴R⁵, NHCOR⁴, CF₃, OCF₃, CN, OR⁴, COOR⁴, (CH₂)ₙCOOR⁴, (CH₂)ₙNR⁴R⁵, -N=C=O ou NHCONR⁴R⁵,
R¹,R²,R³ représentent chacun indépendamment l'un de l'autre un manque, H, Hal, Q, CF₃, NO₂, NR⁴R⁵, CN, COOR⁴ ou NHCOR⁴,
R⁴,R⁵ représentent chacun indépendamment l'un de l'autre H ou Q, ou encore ensemble - CH₂-(CH₂)ₙ-CH₂-,
Q représente un alkyle ayant de 1 à 6 atomes de carbone,
Ph représente un phényle,
X représente O ou S,
Hal représente F, Cl, Br ou I,
n vaut 1, 2 ou 3,
ainsi que leurs sels,
à l'exception des suivants :
4-méthyl-N-(2,1,3-benzothiadiazol-4-yl)-benzénesulfonamide, 4-méthyl-N-(2,1,3-benzothiadiazol-5-yl)-benzènesulfonamide, 4-nitro-N-(2,1,3-benzothiadiazol-4-yl)-benzènesulfonamide, 4-nitro-N-(2,1,3-benzothiadiazol-5-yl)-benzènesulfonamide, 4-amino-N-(2,1,3-benzothiadiazol-4-yl)-benzènesulfon-amide et 4-amino-N-(2,1,3-benzothia-diazol-5-yl)-benzènesulfonamide.

2. a) 5-bromo-2-éthyl-N-(2,1,3-benzothiadiazol-5-yl)-benzènesulfonamide;
b) 2,5-dichloro-N-(2,1,3-benzothiadiazol-5-yl)-benzènesulfonamide;
c) 5-bromo-2-propyl-N-(2,1,3-benzothiadiazol-5-yl)-benzènesulfonamide;
d) 5-diméthylamino-N-(2,1,3-benzothiadiazol-5-yl)-naphtalènesulfonamide;
e) 5-diméthylamino-N-[6-méthyl-(2,1,3-benzo-thiadiazol-5-yl)]-naphtalènesulfonamide;
f) 5-diméthylamino-N-[4-bromo-(2,1,3-benzothiadiazol-5-yl)]-naphtalènesulfonamide;
g) 5-diméthylamino-N-(2,1,3-benzothiadiazol-4-yl)-naphtalènesulfonamide;
h) 5-diméthylamino-N-([1,2,5]-oxadiazole-[3,4-b]-pyridin-6-yl)-naphtalènesulfonamide;
i) 5-diméthylamino-N-(1,2,5-benzoxadiazol-5-yl)-1-naphtalènesulfonamide;
j) 5-diméthylamino-N-(6-bromo-7-méthyl-1,2,5-benzoxadiazol-5-yl)-1-naphtalènesulfonamide;
k) 2-phényl-N-(2,1,3-benzothiadiazol-5-yl)-benzènesulfonamide;
selon la revendication 1.

3. Procédé de préparation de composés de formule I selon la revendication 1 ainsi que de leurs sels, caractérisé en ce que
(a) on fait réagir un composé de formule II 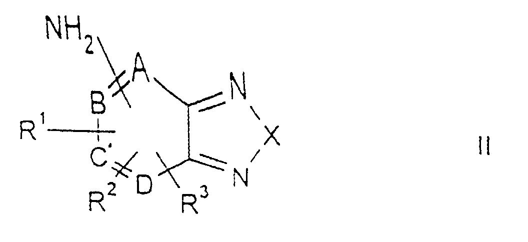
dans laquelle
-A=B-C=D-, R1, R², R³ et X ont les significations données dans la revendication 1, avec un composé de formule III
Ar-SO₂-E III
dans laquelle
E représente Cl, Br, I ou un groupe OH libre ou fonctionnellement modifié réactif et
Ar a la signification indiquée dans la revendication 1,
ou
(b) pour préparer un composé de formule I
dans laquelle
X représente S,
on fait réagir un composé de formule IV 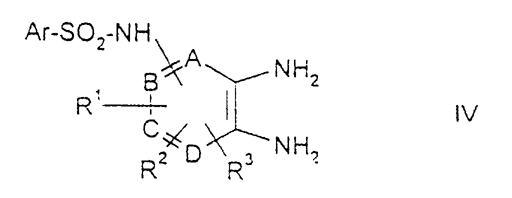
dans laquelle
-A=B-C=D-, Ar, R¹ ,R² et R³ ont les significations données dans la revendication 1, avec du chlorure de thionyle ou un dérivé réactif de ce composé,
ou
(c) pour préparer un composé de formule I,
dans laquelle
X représente O,
on réduit un composé de formule V 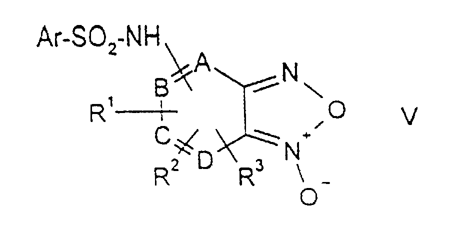
dans laquelle
-A=B-C=D-, Ar, R¹, R² et R³ ont les significations données dans la revendication 1, et/ou
on transforme dans un composé de formule I un ou plusieurs radicaux R¹, R² et/ou R³ en un ou plusieurs radicaux R¹, R² et/ou R³,
en
i) réduisant un groupe nitro en un groupe amino,
ii) remplaçant un substituant brome par un groupe cyano,
iii) hydrolysant un groupe cyano en un groupe carboxy,
iv) estérifiant un groupe carboxy,
v) transformant un groupe amino par une amination réductrice en une amine alkylée, et/ou transformant une base ou un acide de formule I en l'un de leurs sels.

4. Procédé de préparation de préparations pharmaceutiques, caractérisé en ce qu'on met sous une forme d'administration appropriée un composé de formule I selon la revendication 1 et/ou le 4-méthyl-N-(2,1,3-benzothiadiazol-5-yl)-benzènesulfonamide et/ou le 4-nitro-N-(2,1,3-benzothiadiazol-5-yl)-benzènesulfonamide et/ou le 4-amino-N-(2,1,3-benzothiadiazol-5-yl)-benzènesulfonamide et/ou un de leurs sels physiologiquement acceptables avec au moins un support ou additif solide, liquide ou semi-liquide.

5. Préparation pharmaceutique caractérisée en ce qu'elle contient au moins un composé de formule I selon la revendication 1 et/ou le 4-méthyl-N-(2,1,3-benzothiadiazol-5-yl)-benzènesulfonamide et/ou le 4-nitro-N-(2,1,3-benzothiadiazol-5-yl)-benzènesulfonamide et/ou le 4-amino-N-(2,1,3-benzothiadiazol-5-yl)-benzènesulfonamide et/ou un de leurs sels physiologiquement acceptables.

6. Composés de formule I selon la revendication 1 et le 4-méthyl-N-(2,1,3-benzothiadiazol-5-yl)-benzènesulfonamide, le 4-nitro-N-(2,1,3-benzothiadiazol-5-yl)-benzènesulfonamide et le 4-amino-N-(2,1,3-benzothiadiazol-5-yl)-benzènesulfonamide et leurs sels physiologiquement acceptables pour la lutte contre les maladies.

7. Médicament de formule I selon la revendication 1 et le 4-méthyl-N-(2,1,3-benzothiadiazol-5-yl)-benzènesulfonamide, le 4-nitro-N-(2,1,3-benzothiadiazol-5-yl)-benzènesulfonamide et le 4-amino-N-(2,1,3-benzothiadiazol-5-yl)-benzènesulfonamide et leurs sels physiologiquement acceptables comme antagonistes des récepteurs de l'endothéline.

8. Utilisation de composés de formule I selon la revendication 1 et/ou du 4-méthyl-N-(2,1,3-benzothiadiazol-5-yl)-benzènesulfonamide et/ou du 4-nitro-N-(2,1,3-benzothiadiazol-5-yl)-benzène-sulfonamide et/ou du 4-amino-N-(2,1,3-benzothiadiazol-5-yl)-benzénesulfonamide et/ou de leurs sels physiologiquement acceptables pour la préparation d'un médicament.
